(19) Europäisches Patentamt
European Patent Office
Office européen des brevets

(11) EP 1 659 144 B1

(12) EUROPEAN PATENT SPECIFICATION

(45) Date of publication and mention of the grant of the patent:
**21.01.2009 Bulletin 2009/04**

(21) Application number: **04772558.5**

(22) Date of filing: **25.08.2004**

(51) Int Cl.:
***C08J 3/12*** *(2006.01)* ***C08F 2/44*** *(2006.01)*

(86) International application number:
**PCT/JP2004/012602**

(87) International publication number:
**WO 2005/021619 (10.03.2005 Gazette 2005/10)**

# (54) ABSORBENT RESIN PARTICLE, AND ABSORBER AND ABSORBENT ARTICLE EMPLOYING THE SAME

ABSORBIERENDES HARZTEILCHEN UND ABSORBER UND ABSORBIERENDER GEGENSTAND DER DIESES VERWENDET

PARTICULE DE RESINE ABSORBANTE, ABSORBANT CONTENANT CETTE PARTICULE, ET ARTICLE ABSORBANT CONTENANT CETTE PARTICULE

(84) Designated Contracting States:
**BE DE**

(30) Priority: **29.08.2003 JP 2003307803**

(43) Date of publication of application:
**24.05.2006 Bulletin 2006/21**

(73) Proprietor: **San-Dia Polymers, Ltd.**
**Chuo-ku, Tokyo 103-0023 (JP)**

(72) Inventors:
- **SAITO, Takao**
  **c/o San-Dia Polymers, Ltd.**
  **Tokyo 103-0023 (JP)**
- **TAGAWA, Daisuke**
  **c/o San-Dia Polymers, Ltd.**
  **Tokyo 103-0023 (JP)**

(74) Representative: **Vossius & Partner**
**Siebertstraße 4**
**81675 München (DE)**

(56) References cited:
**EP-A- 0 386 897**
**EP-A- 0 450 922**
**EP-A- 0 463 388**
**WO-A-01/45758**
**DE-A1- 10 043 710**
**JP-A- 9 136 966**
**US-A- 4 497 930**
**US-A- 4 732 968**
**US-A- 5 075 373**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 659 144 B1

## Description

The present invention relates to absorbent resin particles, as well as absorbers and absorbent articles in which the absorbent resin particles are employed.

Crosslinked polymers whose amounts of water absorption (absorption amount) are increased by a method of altering and optimizing an amount of a polymerization initiator, polymerization temperature, polymerization concentration, and the like, and by a method of using a chain transfer agent such as thiol have been known (see Patent Document 1 shown below). Furthermore, a large number of crosslinked polymers whose absorption amounts under loading and the like are increased by, for example, a method of treating the vicinity of the surface of polymer particles have been proposed (see Patent Documents 2 and 3 shown below, etc.). Still further, crosslinked polymers whose absorption rates are enhanced by modifying polymer particles so as to be porous have been proposed (see Patent Document 4 shown below, etc.)

Patent document 5 relates to specific absorbent, cross-linked polymers based on partially neutralised, monoethylenically unsaturated monomers that bear acidic groups.

Patent document 6 describes a process for the preparation of water-swellable, water-insoluble polymer particles, the process comprising crosslinking the polymer using at least 2 covalent crosslinking agents, the crosslinking agents comprising a polyvinyl first crosslinking agent and a hydroxyl-containing second crosslinking agent, under conditions such that there is formed a polymer that is substantially uniformly crosslinked, and that has a gel bed permeability of at least $5x\ 10^{-9}\ cm^2$ and an absorption capacity of less than 26 g/g.

A method for forming fine particulate superabsorbent particles into larger particles which comprises: (a) forming a dispersion of a reaction product of a superabsorbent polymer solution polymerization process and fine superabsorbent polymer particles having a particle size of 75 μm or less wherein said dispersion comprises about 0.2 parts to about 4 parts of said fine polymer particles per 95 to 100 parts of said reaction product and wherein said reaction product has a superabsorbent polymer content of from about 10 to about 25 percent; (b) adding from about 4 to 7 parts of water per part of said particles to said dispersion; (c) mixing said water and said dispersion to form a substantially uniform mixture in a second mixing zone; and (d) drying said mixture is described in patent document 7.

Patent document 8 relates to a specific method for the production of a fluid stable aggregate.

Patent document 9 refers to a process for producing a water-absorbent polymer in which the aqueous liquid (I) , which is obtained by mixing in an aqueous medium at least one kind of a water-soluble salt (A) selected from a group of halogenated compounds, sulfates, acetates, and nitrates, which are derived from at least one kind of a polyvalent metal selected from a group of aluminum, calcium, and magnesium, with at least one kind of a water-soluble salt (B) selected from a group of monovalent metal salts and ammonium salts, which are derived from at least one kind of an oxyacid selected from a group of sulfurous acid and thiosulfuric acid, is mixed with a water-absorbent polymer (II) in a proportion of that the water-soluble salt of the polyvalent metal (A) is in a range of 0.1 ~ 10 parts by weight against 100 parts by weight of the water-absorbent polymer (II) and the water-soluble salt of the oxyacid (B) is in a range of 0.1 ~ 10 parts by weight against 100 parts by weight of the water-absorbent polymer (II).

A method for providing an elastomer with water retention properties and water-absorbency comprising mixing the elastomer with finely divided particles of water-absorbent resin having a particle size of 100μm. or finer whose water absorption capacity is not adjusted, in the presence of 10 to 200 parts by weight of water per 100 parts by weight of the said resin and a cross linking agent in an amount of 0.01 to 10 parts by weight per 100 parts by weight of the said resin is disclosed in patent document 10.

Patent document 11 describes a process for granulating a water-absorbent resin, characterized in that 0.000005 to 0.2 parts by weight of a powdery inorganic material is added with agitation in an inert solvent in the presence of 0.1 to 5.0 parts by weight of water and 0.005 to 0.2 parts by weight of a surface active agent to 1 part by weight of a water-absorbent resin containing a carboxylate as a constituent of the polymer, and the water and the inert solvent are removed by distillation.

Patent document 12 relates to a process for producing a highly water-absorbent polymer which is characterized in that a hydrous hydrophilic polymer which has carboxyl groups (or carboxylate groups) and whose water content is adjusted to 10 to 40 wt% is crosslinked by a cross-linking agent having at least two functional groups capable of reacting with the carboxyl group (or the carboxylate group).

Patent document 13 describes modified water-absorptive resin particles obtained by treating (A) water-absorptive resin particles with (B) a silicone surfactant having an HLB of 7 to 18 and a viscosity at 25 ˚C of 10 to 2,000 cSt, provided that the amount of the component (B) is 0.001 to 3 wt.% based on the component (A).

Patent Document 1: JP3(1991)-179008A
Patent Document 2: JP 267529 B
Patent Document 3: European Patent Publication No. 618005A
Patent Document 4: JP 2002-212331 A

Patent document 5: DE 100 43 710
Patent document 6: WO 01/45758
Patent document 7: EP 463 388
Patent document 8: EP 450 922
Patent document 9: EP 386 897
Patent document 10: US 5,075,373
Patent document 11: US 4,732,968
Patent document 12: US 4,497,930
Patent document 13: JP 91-36966

Absorbent articles (disposable diapers, etc.) in which conventional crosslinked polymers are employed have had a problem in that when a user sits down or lies down while wearing such an article, an absorption amount and an absorption rate with respect to a liquid decrease, and as a result, leakage, etc. tend to occur. Therefore, there have been strong demands for absorbent articles that are free from such a problem as leakage, and absorbent resin particles that can be used in such absorbent articles.
In other words, it is an object of the present invention to provide absorbent resin particles that can be used in absorbent articles that exhibit high absorption performances (absorption amount and absorption rate) under any conditions and are substantially free from a problem of leakage.
The inventors of the present invention have investigated earnestly in order to achieve the above-mentioned object and have found that absorbent resin particles having specific structures can solve the above-described problem, thereby to arrive to the present invention.
The absorbent resin particle of the present invention is an absorbent resin particle comprising:

a crosslinked polymer (A) including, as essential constituent units, a water-soluble vinyl monomer (a1) and/or a vinyl monomer (a2) that is formed into the water-soluble vinyl monomer (a1) by hydrolysis, and an internal crosslinking agent (b); and a hydrophobic substance (C) selected from the group consisting of:
a hydrophobic substance (C1) containing a hydrocarbon group;
a hydrophobic substance (C2) containing a hydrocarbon group having a fluorine atom;
a hydrophobic substance (C3) having a polysiloxane structure;

wherein
the absorbent resin particle has a structure such that a part or an entirety of the hydrophobic substance (C) is contained in the inside of each particle of the absorbent resin particle such that each particle of the absorbent resin particle contains a connection (RC) formed with the hydrophobic substance (C), and
the absorbent resin particle being obtainable by either one of methods (1) and (2): the method (1) including forming the connection (RC) and thereafter mixing the hydrophobic substance (C) with a hydrogel of the crosslinked polymer (A) or a polymerization solution of the crosslinked polymer (A); and
the method (2) including forming the connection (RC) while the crosslinked polymer (A) is being produced.
Further, the absorber of the present invention is an absorber that comprises the above-described absorbent resin particle and a fibrous material.
Still further, an absorbent article of the present invention is an absorbent article that is furnished with the above-described absorber.
The absorbent resin particle of the present invention is extremely excellent in the balance among water-retention performance (water-retention amount), a liquid permeation rate and an absorption time of a hydrogel particle (a gel substance formed with an absorbent resin particle that has absorbed liquid), and therefore, provides the dry feeling even after absorption.
Accordingly, when the absorbent resin particle of the present invention is used in an absorbent article such as a disposable diaper, sanitary napkin, etc., the absorbent article thus obtained exhibits excellent absorption performance (absorption amount and absorption rate) under any condition and is substantially free from leakage. Further, the absorbent article also exhibits an excellent feature of preventing absorbed liquid from easily returning even under any condition.

FIG. 1 is a front cross-sectional view schematically illustrating a measuring device for measuring a diffusion absorption amount.
FIG. 2 is a perspective transparent view schematically illustrating a measuring device for measuring a diffusion absorption amount.

First, the crosslinked polymer (A) is described.
The water-soluble vinyl monomer (a1) is not limited particularly, and a vinyl monomer having at least one water-soluble substituent and an ethylenically unsaturated group, or the like can be used as the water-soluble vinyl monomer (a1)

Examples of the water-soluble substituent include a carboxyl (salt) group ($-CO_2M$), a sulfo (salt) group ($-SO_3M$), a group forming an ester of sulfonic acid (salt) (sulfo-oxy (salt) group, $-OSO_3M$), a phosphono (salt) group ($-PO(OM)_2$), a hydroxyl group (-OH), an amino group ($-NR_2$), a group forming an amide (carbamoyl group, $-CONR_2 \cdot Y$), an ammonio group ($-NH_3 \cdot Y$), and secondary, tertiary, or quaternary ammonio groups ($-NR_3 \cdot Y$), and the like. Herein, "carboxyl (salt) group" is intended to mean a carboxyl group or a carboxylate group (a metal carboxylate group or an ammonium caboxylate group). The same is true for other groups.

It should be noted that M represents a hydrogen atom, an alkali metal atom (lithium, sodium, potassium, etc.), an alkali earth metal atom (magnesium, calcium, etc.), or ammonium ($NH_4$); and R represents a hydrogen atom, or a hydrocarbon group having 1 to 7 carbon atoms that may include hetero atoms (methyl, ethyl, propyl, butyl, benzyl, hydroxyethyl, trifluoromethyl, chloroethyl, and the like); Y represents a counter anion to an ammonium cation (examples of the counter anion include chlorine ion, bromine ion, methosulfate ion, and sulfate ion).

Examples of the water-soluble vinyl monomer (a1) include the following (i) anionic vinyl monomers, (ii) nonionic vinyl monomers, and (iii) cationic vinyl monomers.

It should be noted that (i) anionic vinyl monomer may be a salt. Examples of anionic vinyl monomer that is salt include alkali metal salt (sodium salt, potassium salt, and the like), alkali earth metal salt (calcium salt, magnesium salt, and the like), ammonium salt [ammonium salt, tetraalkyl (number of carbon atoms in alkyl: 1 to 8) ammonium salt (tetramethyl ammonium, and the like) etc.], and organic amine salt. Examples of the organic amine composing the organic amine salt include alkyl amine having 1 to 8 carbon atoms, alkanolamine having 2 to 8 carbon atoms, polyalkylene (the number of carbon atoms in alkylene: 1 to 8) polyamine (the number of amino groups: 2 to 10) or derivative of polyalkylene polyamine [a compound that is alkylated with an alkyl group having 1 to 8 carbon atoms, or a compound obtained by adding alkylene oxide having 2 to 12 carbon atoms (average number of added moles per one amino group: 1 to 30 moles) etc.] and the like.

(i) Anionic vinyl monomer

(i-1) For the vinyl monomer having a carboxyl (salt) group ($-CO_2M$), carboxylic acid (salt) containing a vinyl group having 3 to 30 carbon atoms and the like is used. Examples thereof include unsaturated mono-carboxylic acid ((meth)acrylic acid, (meth)acrylate, crotonic acid, cinnamic acid, etc.); unsaturated dicarboxylic acid (maleic acid, salt of maleic acid, fumaric acid, citraconic acid, itaconic acid, etc.); and monoalkyl (the number of carbon atoms: 1 to 8) ester of the unsaturated dicarboxylic acid (monobutyl maleate, monobutyl fumarate, ethylcarbitol monoester of maleic acid, ethylcarbitol monoester of fumaric acid, monobutyl citraconate, monoglycol itaconate, etc.).

(i-2) For vinyl monomer having a sulfo (salt) group ($-SO_3M$), a sulfonic acid (salt) containing a vinyl group having 2 to 30 carbon atoms or the like is used. Examples of the same include aliphatic or aromatic vinyl sulfonic acid (vinyl sulfonic acid, (meth)allyl sulfonic acid, styrene sulfonic acid, α-methyl stylene sulfonic acid, etc.); alkyl sulfonic acid containing (meth)acryloyl group ((meth)acryloxy propyl sulfonic acid, 2-hydroxy-3-(meth)acryloxy propyl sulfonic acid, 2-(meth)acryloylamino-2,2-dimethyl ethane sulfonic acid, 3-(meth)acryloxy ethane sulfonic acid, 2-(meth)acrylamide-2-methylproparie sulfonic acid and 3-(meth)acrylamide-2-hydroxypropane sulfonic acid, etc.); and alkyl (the number of carbon atoms: 3 to 18) (meth)allyl sulfosuccinate. In the present invention, the term such as "(meth) acryl..." or "(meth)allyl..." is intended to mean "acryl..." or "methacryl..." for "(meth)acryl ...", and "allyl..." or "methallyl" for "(meth)allyl ...", respectively.

(i-3) Examples used as vinyl monomer having a group forming ester of sulfonic acid (salt) (sulfo-oxy group, $-OSO_3M$) include: sulfuric-acid esterified hydroxyalkyl (the number of carbon atoms: 2 to 6) (meth)acrylate [for example, sulfuric-acid esterified hydroxyethyl (meth)acrylate]; and sulfuric-acid esterified poly (polymerization degree: 2 to 30) oxyalkylene (the number of carbon atoms of alkylene group is 2 to 4, and the polymerization form is single or random and/or block) mono (meth)acrylate [for example, sulfuric-acid esterified poly (polymerization degree: 5 to 15) oxypropylene monomethacrylate]; and compounds expressed by general formulae (5), (6) or (7).

$$\begin{array}{l} CH_2=CHCH_2OCH_2\underset{\displaystyle (OA)_nOSO_3M}{\underset{|}{CH}}-CH_2O-Ar-R \end{array} \qquad (5)$$

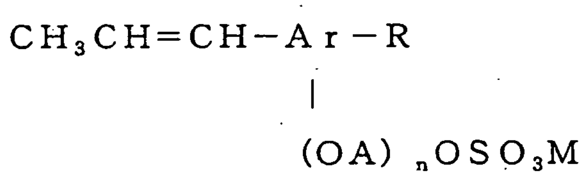

(6)

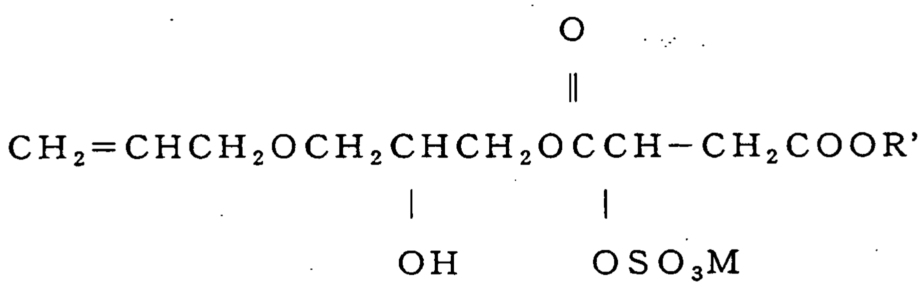

(7)

In the above-mentioned general formulae (5) to (7), R represents a hydrogen atom or an alkyl group having 1 to 15 carbon atoms. R'represents a hydrogen atom; an alkyl group having 1 to 15 carbon atoms, which may be substituted by a fluorine atom; an alkali metal atom (lithium, sodium, potassium, etc.); an alkali earth metal atom (magnesium, calcium, etc.); or ammonium. OA represents oxyalkylene group having 2 to 4 carbon atoms; and when n is 2 or more, two or more OAs may be the same or different; and when the OAs are different, they may be random or block or the mixture thereof; Ar represents a benzene ring; M represents a hydrogen atom, an alkali metal atom, an alkali earth metal atom, or ammonium; and n denotes an integer of 1 to 50.

Examples of an alkyl group include methyl, ethyl, propyl, t-butyl, 2-ethylhexyl, dodecanyl, and pentadecanyl.

Examples of an alkyl group that may be substituted by a fluorine atom include methyl, ethyl, t-butyl, 2-ethylhexyl, pentadecanyl, trifluoromethyl, and pentafluoroethyl, etc.

Examples of an oxyalkylene group include oxyethylene, oxypropylene, and oxybutylene, etc.

(i-4) Examples of a vinyl monomer having a phosphono (salt) group ($-PO(OM)_2$) include phosphoric monoester of hydroxyalkyl (the number of carbon atoms: 2 to 6) (meth)acrylate [for example, monophosphate of hydroxyethyl (meth)acrylate]; phosphoric diester of hydroxyalkyl (the number of carbon atoms: 2 to 6) (meth)acrylate [for example, phenyl-2-acryloyloxyethyl phosphate]; and (meth)acrylic acid alkyl (the number of carbon atoms: 2 to 6) phosphonic acid [for example, 2-acryloyloxyethyl phosphonic acid] etc.

(ii) Nonionic vinyl monomer

(ii-1) Examples of vinyl monomer having a hydroxyl group (-OH) include monoethylenically unsaturated alcohol having the 3 to 15 carbon atoms [for example, (meth)allyl alcohol and (meth)propenyl alcohol]; and monoethylenically unsaturated carboxylic acid ester of polyol having 2 to 6 valences (examples of the polyol include alkylene glycol having 2 to 20 carbon atoms, glycerin, sorbitan, diglycerine, pentaerythritol, polyalkylene (number of carbon atoms: 2 to 4) glycol (weight-average molecular weight: 100 to 2000), etc.), and monoethylenically unsaturated ether of the polyol.

Specifically, examples of the same include hydroxyethyl (meth)acrylate, hydroxypropyl (meth)acrylate, triethylene glycol (meth)acrylate, and poly-oxyethylene-oxypropylene (random and/or block, weight-average molecular weight: 100 to 2000) mono(meth)allyl ether (a hydroxyl group at an end may be etherified or esterified with an alkyl (methyl, ethyl or butyl, etc.) group having 1 to 4 carbon atoms) or with a saturated fatty acid (acetic acid, propionic acid, etc.) having 2 or 3 carbon atoms.

(ii-2) Examples of vinyl monomer having a group forming an amide (carbamoyl group, $-CONR_2$) include: (meth) acrylamide; N-alkyl (the number of carbon atoms: 1 to 8) (meth)acrylamide [for example, N-methyl acrylamide]; N, N-dialkyl (the number of carbon atoms in alkyl: 1 to 8) acrylamide [for example, N,N-dimethyl acrylamide, N,N-di-n-, or i-propyl acrylamide]; N-hydroxyalkyl (the number of carbon atoms: 1 to 8) (meth)acrylamide [for example, N-methylol (meth)acrylamide, or N-hydroxyethyl (meth)acrylamide]; and N,N-dihydroxyalkyl (the number of carbon atoms: 1 to 8) (meth)acrylamide [for example, N,N-dihydroxyethyl (meth)acrylamide].
As a vinyl monomer having a group forming an amide, other than the above, vinyl lactam having 5 to 10 carbon atoms (for example, N-vinylpyrrolidone) or the like may be used.
(ii-3) Examples of vinyl monomer having an amino group ($-NR_2$) include an amino group-containing ester of monoethylenically unsaturated mono- or di-carboxylic acid, and amino group-containing amide of monoethylenically unsaturated mono- or di-carboxylic acid.

As the amino group-containing ester of monoethylenically unsaturated mono- or di-carboxylic acid, the following can be used: dialkyl (the number of carbon atoms in alkyl: 1 to 8) amino alkyl (the number of carbon atoms: 2 to 10) (meth)acrylate; di(hydroxyalkyl) (the number of carbon atoms in alkyl: 1 to 8) amino alkyl (the number of carbon atoms: 2 to 10) ester; and morpholino alkyl (the number of carbon atoms: 1 to 8) ester. Examples of the same include: dimethyl aminoethyl (meth)acrylate; diethyl amino (meth)acrylate; morpholino ethyl (meth)acrylate; dimethyl amino ethyl fumarate; and dimethyl amino ethyl malate.

As the amino group-containing amide of monoethylenically unsaturated mono- or di-carboxylic acid, monoalkyl (the number of carbon atoms: 2 to 10) (meth)acrylamide or the like can be used, examples of which include dimethyl amino ethyl (meth)acrylamide, and diethyl amino ethyl (meth)acrylamide.

As a vinyl monomer having an amino group, other than the above, vinylpyridine (for example, 4-vinylpyridine, or 2-vinylpyridine) can be used.

(iii) Cationic vinyl monomer

(iii-1) Examples of vinyl monomer having an ammonio group ($-NH_3 \cdot Y$) include:

ammonio group-containing ester of monoethylenically unsaturated mono- or di-carboxylic acid [for example, ammonio alkyl (the number of carbon atoms of 2 to 10) (meth)acrylate {ammonio ethyl (meth)acrylate-chloride, etc.}]; and
ammonio group containing amide of monoethylenically unsaturated mono- or di-carboxylic acid [for example, ammonio alkyl (the number of carbon atoms of 2 to 10) (meth)acrylamide {ammonio ethyl(meth)acrylamide methosulfate, etc.}].

(iii-2) Examples of vinyl monomer having a mono- or di-alkyl ammonio group ($-NRH_2 \cdot Y$ or $-NR_2 \cdot H \cdot Y$) include:

alkyl ammonio group-containing ester of monoethylenically unsaturated mono- or di-carboxylic acid [monoalkyl (the number of carbon atoms in alkyl: 1 to 4) ammonio alkyl (the number of carbon atoms: 2 to 10) (meth) acrylate {methyl ammonio ethyl (meth)acrylate·chloride, t-butyl ammonio ethyl (meth)acrylate·methosulfate, etc.}, dialkyl (the number of carbon atoms: 1 to 4) ammonio alkyl (the number of carbon atoms: 2 to 10) (meth) acrylate {dimethyl ammonio ethyl (meth)acrylate·chloride, methyl t-butyl ammonio ethyl (meth)acrylate·bromide, etc.}, etc.]; and
alkyl ammonio group-containing amide of monoethylenically unsaturated mono- or di-carboxylic acid [monoalkyl (the number of carbon atoms: 1 to 4) ammonio alkyl (the number of carbon atoms: 2 to 10) (meth)acrylamide {methyl ammonio ethyl (meth)acrylamide·chloride, butyl ammonio ethyl(meth)acrylamide·chloride, etc.}, dialkyl (the number of carbon atoms in alkyl: 1 to 4) ammonio alkyl (the number of carbon atoms: 2 to 10) (meth) acrylamide {dimethyl ammonio ethyl (meth)acrylamide·chloride and methyl propyl ammonio ethyl (meth)acrylamide·methosulfate, etc.}, etc.].

(iii-3) Examples of vinyl monomer having a trialkyl ammonio group ($-NR_3 \cdot Y$) include a vinyl monomer obtained by quaternizing the vinyl monomer having an amino group by an alkylating agent having 1 to 8 carbon atoms (a quaternization agent such as methyl chloride, dimethyl sulfuric acid, benzil chloride, dimethyl carbonate, or the like) {for example, trimethyl ammonio ethyl (meth)acrylate·chloride, methyl diethyl ammonio ethyl (meth)acrylate·methosulfate, trimethyl ammonio ethyl malate·chloride, trimethyl ammonio ethyl (meth)acrylamide·chloride, diethyl benzil ammonio ethyl (meth)acrylamide·chloride} and the like. Other than the above, N-vinylpyridinium salt (N-vinylpyridinium·chloride, N-methyl-2-vinylpyridinium-chloride, etc.) also may be used.

A hydrophilic-lipophilic balance (HLB) value of water-soluble vinyl monomer is preferably 10 to 20, more preferably 11.5 to 20, and particularly preferably 13 to 20.

It should be noted that the HLB value in the present invention is a value calculated according to HLB by Davis (Dr. Takehiko Fujimoto, "Shin Kaimen Kassei-zai Nyumon" (Japanese edication), third printing in August 1992, SANYO CHEMICAL INDUSTRIES, LTD, page 132, corresponding to English edition "New Introduction to Surface Active Agents" Copyright 1985, page 132.)

There is no particular limitation to the vinyl monomer (a2) that is formed into the water-soluble vinyl monomer (a1) by hydrolysis. However, a vinyl monomer having at least one hydrolyzable substituent that becomes a water-soluble substituent upon hydrolysis, or the like, can be used as the vinyl monomer (a2).

Examples of a hydrolyzable substituent include a group forming acid anhydride (1,3-oxo-oxapropylene group, -COO-CO-), a group forming an ester linkage (alkyloxycarbonyl, vinyloxycarbonyl, allyloxycarbonyl, or propenyloxycarbonyl, -COOR), and a cyano group.

It should be noted that R denotes an alkyl group having 1 to 3 carbon atoms (methyl, ethyl and propyl), a vinyl group, an allyl group and propenyl group.

As the vinyl monomer having a group forming acid anhydride, dicarboxylic acid anhydride having 4 to 20 carbon atoms or the like is used. Examples thereof include maleic anhydride, itaconic anhydride, citraconic anhydride, and the like.

Examples of vinyl monomer having a group forming ester include lower alkyl (the number of carbon atoms: 1 to 3) ester of monoethylenically unsaturated carboxylic acid [methyl (meth)acrylate, ethyl (meth)acrylate, etc.], and ester of monoethylenically unsaturated alcohol [vinyl acetate, (meth)allyl acetate, etc.].

Examples of vinyl monomer having a cyano group include a nitrile compound containing a vinyl group having 3 to 6 carbon atoms [(meth)acrylonitrile, 5-hexene nitrile, etc.].

Hydrolysis of the hydrolyzable substituent may be carried out at any time of during polymerization, after polymerization, or at both occasions. However, from the viewpoint of the molecular weight of the resultant crosslinked polymer, hydrolysis preferably is carried out after polymerization.

In the case where either the vinyl monomer (a1) or the vinyl monomer (a2) is used as a constituent unit, either of them may be used singly as the constituent unit, or they may be used in combination of two kinds or more as the constituent unit if necessary. The same applies in the case where both of the vinyl monomers (a1) and (a2) are used as the constituent units.

Among them, vinyl monomer is preferably a water-soluble monomer (a1), more preferably anionic vinyl monomer, particularly preferably a vinyl monomer having a carboxyl (salt) group, a sulfo (salt) group, an amino group, a group forming amide, an ammonio group, or a mono-, di- or tri-alkyl ammonio group, even more preferably a vinyl monomer having a carboxyl (salt) group or a group forming amide, further particularly preferably (meth)acrylic acid (salt) or (meth)acrylamide, even further particularly preferably (meth)acrylic acid (salt), and most preferably acrylic acid (salt).

In the case where the water-soluble vinyl monomer (a1) and the vinyl monomer (a2) are used as constituent units, the ratio of the contents thereof (a1/a2) by weight preferably is 75/25 to 99/1, more preferably 85/15 to 95/5, particularly preferably 90/10 to 93/7, and most preferably 91/9 to 92/8. With the ratio in such a range, a further improved absorption performance (particularly absorption amount and absorption rate, etc.) is obtained.

As the vinyl monomer used as a constituting unit of a crosslinked polymer (A), other than the vinyl monomers (a1) and/or (a2), vinyl monomer (a3) copolymerizable with vinyl monomer (a1) and/or (a2) can be used as the constituent unit.

As the other vinyl monomer (a3) copolymerizable therewith, hydrophobic vinyl monomer, etc. can be used, but it is not limited thereto.

Examples of the other vinyl monomer (a3) include the following vinyl monomers (i) to (iii):

(i) aromatic ethylenic monomer having 8 to 30 carbon atoms: a styrene such as styrene, $\alpha$-methyl styrene, vinyltoluene, and hydroxy styrene.; vinylnaphthalene; and, a halogen substituted styrene such as dichlorostyrene;
(ii) aliphatic ethylenic monomer having 2 to 20 carbon atoms: alkene [ethylene, propylene, butene, isobutylene, pentene, heptene, diisobutylene, octane, dodecene, octadecene, and the like]; and alkadiene [butadiene, isoprene, and the like] etc.
(iii) alicyclic ethylenic monomer having 5 to 15 carbon atoms: monoethylenically unsaturated monomer [pinene, limonene, indene, and the like]; polyethylenic vinyl polymerizable monomer [cyclopentadiene, bicyclopentadiene, ethylidene norbornane, and the like] etc.

In the case where the other vinyl monomer (a3) that is copolymerizable is used as the constituent unit, the content of (a3) is preferably 0.01 percent by weight (wt%) to 5 wt%, more preferably 0.05 wt% to 3 wt%, particularly preferably 0.08 wt% to 2 wt% and most preferably 0.1 wt% to 1.5 wt% with respect to the total weight of the vinyl monomers (a1) and (a2). With the content in such a range, a further improved absorption performance (particularly absorption amount and absorption rate, etc.) can be obtained.

Examples of the internal crosslinking agent (b) include: an internal crosslinking agent (b1) having two or more

ethylenically unsaturated groups; an internal crosslinking agent (b2) having at least one functional group capable of reacting with the water soluble substituent of the water-soluble vinyl monomer (a1) and/or the water soluble substituent generated by hydrolysis of the vinyl monomer (a2) and having at least one ethylenically unsaturated group; and an internal crosslinking agent (b3) having at least two functional groups capable of reacting with the water soluble substituent of the water-soluble vinyl monomer (a1) and/or the water soluble substituent generated by hydrolysis of the vinyl monomer (a2), and the like.

(i) As the internal crosslinking agent (b1) having two or more of ethylenically unsaturated groups, bis(meth)acrylamide having 8 to 12 carbon atoms, polyol poly(meth)acrylate having 2 to 10 carbon atoms, polyallylamine having 2 to 10 carbon atoms, and polyol poly(meth)allyl ether having 2 to 10 carbon atoms, or the like is used. Examples thereof include N,N'-methylenebis (meth)acrylamide, ethylene glycol di(meth)acrylate, poly (polymerization degree: 2 to 5) ethylene glycol di(meth)acrylate, propylene glycol di(meth)acrylate, glycerin (di- or tri-) acrylate, trimethylolpropane triacrylate, diallyl amine, triallyl amine, triallyl cyanurate, triallyl isocyanurate, tetraallyloxy ethane, pentaerythritol diallyl ether, pentaerythritol triallyl ether, pentaerythritol tetraallyl ether, and diglycerin di(meth)acrylate and the like
(ii) As the internal crosslinking agent (b2) having at least one functional group capable of reacting with the water soluble substituent of the water-soluble vinyl monomer (a1) and/or the water soluble substituent generated by hydrolysis of the vinyl monomer (a2) and having at least one ethylenically unsaturated group, an ethylenically unsaturated compound having an epoxy group and having 6 to 8 carbon atoms, an ethylenic unsaturated compound having a hydroxyl group and having 4 to 8 carbon atoms, or an ethylenically unsaturated compound having an isocyanato group and having 4 to 8 carbon atoms is used. Examples thereof include glycidyl (meth)acrylate, N-methylol (meth)acrylamide, hydroxyethyl (meth)acrylate, isocyanato ethyl (meth)acrylate, and the like.
(iii) As the internal crosslinking agent (b3) having at least two functional groups capable of reacting with the water soluble substituent of the water-soluble vinyl monomer (a1) and/or water soluble substituent generated by hydrolysis of the vinyl monomer (a2), polyol, polyglycidyl, polyamine, polyaziridine, polyisocyanate, or the like can be used, which are described in JP 58(1983)-180233A (corresponding US Patent 4666983) and JP 59(1984)-189103A. Examples of polyglycidyl compound include ethylene glycol diglycidyl ether, and glycerin diglycidyl ether. Examples of polyamine compound include ethylene diamine, diethylene triamine, triethylene tetramine, tetraethylene pentamine, pentaethylene hexamine, and polyethylene imine. Examples of polyaziridine compound include "CHEMITITE PZ-33" (trade name) {2,2-bishydroxymethyl butanol-tris (3-(1-aziridinyl) propynate)}, "CHEMITITE HZ-22" (trade name) {1,6-hexamethylene diethylene urea}, and "CHEMITITE DZ-22" (trade name) {diphenylmethane-bis-4,4'-N, N' diethylene urea} (these are trade names of the products of Nippon Shokubai Co., Ltd), etc. Examples of polyisocyanate compound include 2,4-tolylene diisocyanate and hexamethylene diisocyanate.

These internal crosslinking agents may be used singly or in combination of two or more kinds.
Among these, from the viewpoint of the absorption performance (absorption amount, absorption rate, etc. in particular), the internal crosslinking agent (b) preferably is the internal crosslinking agnet (b1) having two or more ethylenically unsaturated groups, more preferably polyol poly(meth)allyl ether having 2 to 10 carbon atoms, particularly preferably triallyl cyanurate, triallyl isocyanurate, tetraallyloxy ethane, pentaerythritol triallyl ether, and most preferably pentaerythritol triallyl ether.
The content of the internal crosslinking agent (b) is preferably 0.001 wt% to 5 wt%, more preferably 0.002 wt% to 2 wt%, and particularly preferably 0.003 wt% to 1.6 wt% with respect to the total weight of the vinyl monomer (a1) and/or (a2) as well as the other vinyl monomer (a3) used if necessary. With the content thereof in this range, the absorption performance (particularly absorption amount and absorption rate, etc.) is enhanced further.
The crosslinked polymer (A) further may contain additives that will be described later (antiseptic agent, fungicide, antibacterial agent, antioxidant, ultraviolet absorber, coloring agent, perfuming agent, deodorizer and organic fibrous material, and the like) as required.
In the case where the crosslinked polymer (A) contains such an additive, the content of the additive preferably is in a range of 0.001 wt% to 10 wt%, more preferably 0.01 wt% to 5 wt%, particularly preferably 0.05 wt% to 1 wt%, and most preferably 0.1 wt% to 0.5 wt% based on the weight of the crosslinked polymer (A).
Regarding the polymerization of the crosslinked polymer (A), a conventionally known method can be used. Examples of the polymerization method include a solution polymerization method, an emulsion polymerization method, a suspension polymerization method, and a reversed-phase suspension polymerization method. The form of the polymerization solution upon polymerization may be in a thin film form or a spray form. As the method for controlling polymerization, an adiabatic polymerization method, a temperature control polymerization method, an isothermal polymerization method, or the like can be used.
When the suspension polymerization method or the reversed-phase suspension polymerization method is employed for the polymerization method, a conventionally known dispersing agent (sucrose ester, phosphate ester, sorbitan ester, etc.), a conventionally known protective colloid (poval, α-olefin-maleic anhydride copolymer, polyethylene

oxide, etc.) can be used as required. Furthermore, when the reversed-phase suspension polymerization method is used, polymerization can be carried out by using a conventionally known solvent such as cyclohexane, normal hexane, normal heptane, toluene, xylene, etc.

Among the polymerization methods, a solution polymerization method is preferable. An aqueous solution polymerization method is particularly preferable, since organic solvent and the like, is not required and it is advantageous in production cost.

For the polymerization for forming the crosslinked polymer (A), a polymerization initiator can be used. The polymerization initiator is not particularly limited and a conventionally known one can be used. As the polymerization initiator, (i) an azo type initiator, (ii) a peroxide type initiaor, (iii) a redox type initiator, or (iv) an organic halide compound initiator can be used.

(i) Examples of the azo type initiator include azobisisobutyronitrile, azobiscyano valeric acid and the salt thereof, 2, 2'-azobis (2-amidinopropane) hydrochloride, and 2, 2'-azobis (2-methyl-N-(2-hydroxyethyl) propionamide, and the like.

(ii) Examples of the peroxide type initiator include inorganic peroxide [for example, hydrogen peroxide, ammonium persulfate, potassium persulfate, sodium persulfate, and the like], organic peroxide [for example, benzoyl peroxide, di-t-butyl peroxide, cumene hydroperoxide, succinic acid peroxide, di(2-ethoxyethyl) peroxy dicarbonate, etc.] and the like.

(iii) As the redox type initiator, a combination of at least one reductant and at least one oxidizer can be used. Examples of the reductant include sulfites of alkali metals, bisulfites of alkali metals, ammonium sulfite, ammonium bisulfite, ferric chloride, ferric sulfate and ascorbic acid. Examples of the oxidizer include persulfates of alkali metals, ammonium persulfate, hydrogen peroxide, and organic peroxide.

(iv) Used as the organic halogen compound initiator is an organic halogen compound having 1 to 10 halogen atoms and 1 to 15 carbon atoms, selected from the group consisting of alkyl halide, halogenated alkyl phenyl ketone, halogenated alkyl carboxylic acid, and halogenated alkyl carboxylic acid alkyl ester. Examples of such an organic halide compound include tetrachloromethane, trichlorobromomethane, trichloroiodomethane, dichloro methylphenyl ketone, 1-bromo-1-methylethylcarboxylic acid, and 1-bromo-1-methylethylcarboxylic acid alkyl ester having an alkyl group with 1 to 12 carbon atoms (for example, methyl 1-bromo-1-methylethylcarboxylate, ethyl 1-bromo-1-methylethyl carboxylate, octyl 1-broino-1-methylethyl carboxylate, and lauryl 1-bromo-1-methylethyl carboxylate).

Among them, (i) azo type initiator, (ii) peroxide type initiator, and (iii) redox type initiator are preferable. It is more preferable to use either (i) azo type initiator, or a combination of (ii) peroxide initiator and (iii) redox type initiator.

In the case where a polymerization initiator is used, the amount of the polymerization initiator to be used is preferably in a range of 0.005 wt% to 0.5 wt%, more preferable 0.007 wt% to 0.4 wt%, and particularly preferably 0.009 wt% to 0.3 wt%, based on the total weight of the vinyl monomer (a1) and/ or (a2), the other vinyl monomer (a3) used if necessary, and the internal crosslinking agent (b).

Upon the polymerization for forming the crosslinked polymer (A), the polymerization may be carried out in a state in which a hydrophobic substance (C) or a material (D), which will be described later, is dissolved or dispersed in the vinyl monomer (a1) and/or (a2), the vinyl monomer (a3), and the internal crosslinking agent (b), or alternatively in a solvent.

The hydrogel obtained by polymerization (containing the crosslinked polymer (A) and water) can be crushed as required. The size of each piece of the crushed hydrogel (maximam diameter) preferably is in a range of 50 $\mu$m to 10 cm, more preferably in a range of 100 $\mu$m to 2 cm, and particularly preferably in a range of 1 mm to 1 cm. With the size in such a range, a further improved dryness can be obtained in the drying process.

The crushing can be carried out by a conventionally known method, by using a conventional device such as a bexmill, a rubber chopper, a pharmamill, a mincing machine, an impact crusher, a roll crusher, or the like.

In the case where a solvent (containing water) is used in the polymerization for forming the crosslinked polymer (A), the solvent can be removed by distillation after the polymerization.

In the case where an organic solvent is contained in the solvent, the content of the organic solvent after the removal by distillation preferably is in a range of 10 wt% to 0.01 wt%, more preferably in a range of 5 wt% to 0.05 wt%, particularly preferable in a range of 3 wt% to 0.1 wt%, and most preferably in a range of 1 wt% to 0.5 wt% based on the weight of the crosslinked polymer (A). With the content in such a range, a further improved absorption property (particularly water-retention amount) of the absorbent resin particle is obtained.

In the case where the solvent contains water, the content of moisture after the distillation is preferably 0 wt% to 20 wt%, more preferably 0 wt% to 10 wt%, particularly preferably 0 wt% to 5 wt%, and most preferably 0 wt% to 2 wt% based on the weight of the crosslinked polymer (A). With the water content in such a range, a further improved absorption performance (particularly water-retention amount) and a further improved handling property after drying (powder fluidity of the crosslinked polymer particles, etc.) are obtained.

It should be noted that the content of the organic solvent and the content of moisture can be determined by subtracting the weight of the crosslinked polymer (A) after heating from the weight of the same before heating. The heating is carried out by using an infrared water content measuring machine ("JE400", manufactured by KETT Co.: 120±5°C, 30 minutes, atmospheric humidity before heating: 50±10 %RH, lamp specification 100V, 40W).

The method for removing the solvent by distillation may be a conventional method, for example, a method of removal by distillation (drying) by using a heated air flow at a temperature of 80°C to 230°C, a thin film drying method by using a drum dryer that is heated at 100°C to 230°C, a reduced pressure drying method (heating may be applied), a freeze drying method, a drying method using infrared ray, decantation, or filtration.

Furthermore, the crosslinked polymer (A) after drying can be pulverized.

When the crosslinked polymer is pulverized, a weight-average particle diameter of the crosslinked polymer after pulverization is preferably 100 μm to 800 μm, more preferably 200 μm to 500 μm, and particularly preferably 300 μm to 400 μm. With the weight-average particle size in such a range, a further improved handling property (powder fluidity of the crosslinked polymer particle, etc.) after being pulverized is obtained.

The weight-average particle diameter is calculated as follows. The particle size distribution of the crosslinked polymer is plotted on a logarithm probability paper in which the traverse axis shows particle diameter and the longitudinal axis shows content by weight. The diameter of particles accounting for 50 wt% of the total weight of the particles is defined as the weight-average particle diameter.

Particle size distribution is measured according to JIS Z8815-1994 as follows. Sieves having an inner diameter of 150 mm, a depth of 45 mm, and mesh-openings of 710 μm, 500 μm, 300 μm, 150 μm and 106 μm respectively are superposed on one another in an opening-size-descending order from the top. 50g of a sample to be measured is placed on the sieve having the largest opening, i.e., 710 μm, that is located at the top and subjected to sieving for 10 minutes by using a vibrating machine. The weight of the sample remaining in each sieve is measured, and the percentage by weight of the portion of the sample remaining on each sieve with respect to the initial weight of the sample is calculated.

The smaller the content of fine particles is, the better the absorption performance is. Preferably, the content of fine particles having the particle diameter of 106 μm or less is 3 wt% or less with respect to the total weight of the particles, and more preferably, the content of fine particles having the particle diameter of 150 μm or less is 3 wt% or less with respect to the total weight of the particles.

The content of the fine particles can be obtained by using a plot generated when the above-mentioned weight-average particle diameter is obtained.

The pulverizing method is not particularly limited, and a conventional device such as a hammer crusher, an impact crusher, a roll crusher, a jet streaming crusher, etc. can be used.

The obtained pulverized product is classified with a sieve if necessary so that the particle size is adjusted.

The shape of the crosslinked polymer particles is not particularly limited. Examples of the shape include an indeterminate crushed shape, a scale shape, a pearl shape, and a rice grain shape. Among these shapes, the indeterminate crushed shape is preferable because the particles in such a shape easily can be entangled with fibrous materials when used for a disposable diaper, etc., and there is little possibility of the crosslinked polymer particles falling off from the fibrous materials.

As the hydrophobic substance (C), a hydrophobic substance (C1) containing a hydrocarbon group, a hydrophobic substance (C2) containing a hydrocarbon group having a fluorine atom, and a hydrophobic substance (C3) having a polysiloxane structure can be used. Among these, the hydrophobic substance (C3) having a polysiloxane structure is preferred with a view to improving the leakage resistance of an absorbent article.

Examples of the hydrophobic substane (C1) containing a hydrocarbon group include polyolefin resins, polyolefin resin derivatives, polystyrene resins, polystyrene resin derivatives, waxes, long-chain fatty acid ester resins, long-chain fatty acids, and a mixture of two or more of the same.

As the polyolefin resin, a polymer having a weight-average molecular weight of 1000 to 1000000 that includes an olefin having 2 to 4 carbon atoms such as ethylene, propylene, isobutylene, isoprene, or the like as an essential constituent monomer (the content of the olefin is at least 50 wt% based on a weight of the polyolefin resin), or the like, can be used. Examples of the same include polyethylene, polypropylene, polyisobutylene, poly(ethylene-isobutylene), and isoprene.

As the polyolefin resin derivative, for example, a polymer having a weight-average molecular weight in a range of 1000 to 1000000 and being obtained by introducing a carboxyl group (-COOH) or 1,3-oxo-2-oxapropylene (-COOCO-) into a polyolefin resin (acid-modified polyolefin resin) can be used. Examples of the same include heat degraded polyethylene, heat degraded polypropylene, maleic acid-modified polyethylene, chlorinated polyethylene, maleic acid-modified polypropylene, ethylene-acrylic acid copolymer, ethylene-maleic anhydride copolymer, isobutylene-maleic anhydride copolymer, maleated polybutadiene, ethylene-vinyl acetate copolymer, and maleated ethylene-vinyl acetate copolymer. It should be noted that in the case of a copolymer with ethylene or butylene, the content of ethylene or butylene preferably is 60 mol% to 99.9 mol% based on the number of moles of the constituent monomer.

As the polystyrene, a polymer having a weight-average molecular weight in a range of 1000 to 1000000 or the

like can be used.

As the polystyrene resin derivative, for example, a polymer having a weight-average molecular weight of 1000 to 1000000 and including styrene as an essential constituent monomer (the content of the styrene is at least 50 wt% based on a weight of the polystyrene derivative) can be used. Examples of the same include styrene-maleic anhydride copolymer, styrene-butadiene copolymer, and styrene-isobutylene copolymer.

As the wax, for example, a wax having a melting point of 50°C to 200°C can be used. Examples of the same include paraffin wax, beeswax, carbana wax, and tallow.

As the ester of a long-chain fatty acid, for example, ester of an fatty acid having 8 to 25 carbon atoms and an alcohol having 1 to 5 carbon atoms can be used. Examples of the same include methyl laurate, ethyl laurate, methyl stearate, ethyl stearate, methyl oleate, ethyl oleate, glycerol monolaurate, glycerol monostearate, glycerol monooleate, pentaerythritol monolaurate, pentaerythritol monostearate, pentaerythritol monooleate, sorbitol monolaurate, sorbitol monostearate, solbitol monooleate, and tallow.

As the long-chain fatty acid, an fatty acid having 8 to 25 carbon atoms or the like can be used. Examples of the same include lauric acid, stearic acid, oleic acid, dimer acid, and behenic acid.

Among the examples of the hydrophobic substance (C1) containing a hydrocarbon group, polyolefin resins, waxes, and long-chain fatty acid esters are preferred with a view to improving the leakage resistance of an absorbent article to be obtained. Among these, the polyolefin resins and waxes are further preferred. Among these, waxes are particularly preferred. Among these, paraffin wax is most preferred.

Examples of the hydrophobic substance (C2) containing a hydrocarbon group having a fluorine atom include perfluoroalkanes, perfluoroalkenes, perfluoroaryls, perfluoroalkyl ethers, perfluoroalkyl carboxylic acids, perfluoroalkyl alcohols, and a mixture of two or more of the same.

As a perfluoroalkane, for example, an alkane having 4 to 42 fluorine atoms and 1 to 20 carbon atoms can be used. Examples of the same include trifluoromethane, pentafluoroethane, pentafluoropropane, heptafluoropropane, heptafluorobutane, nonafluorohexane, tridecafluorooctane, and heptadecafluorododecane.

As a perfluoroalkene, for example, an alkene having 4 to 42 fluorine atoms and 2 to 20 carbon atoms can be used. Examples of the same include trifluoroethylene, pentafluoropropene, trifluoropropene, heptafluorobutene, nonafluorohexene, tridecafluorooctene, and heptadecafluorododecene.

As a perfluoroaryl, for example, an aryl compound having 4 to 42 fluorine atoms and 6 to 20 carbon atoms can be used. Examples of the same include trifluorobenzene, pentafluorotoluene, trifluoronaphthalene, heptafluorobenzene, nonafluoroxylene, tridecafluorooctylbenzene, and heptadecafluorododecylbenzene.

As a perfluoroalkyl ether compound, for example, an ether having 2 to 82 fluorine atoms and 2 to 40 carbon atoms can be used. Examples of the same include ditrifluoromethyl ether, dipentafluoroethyl ether, dipentafluoropropyl ether, diheptafluoropropyl ether, diheptafluorobutyl ether, dinonafluorohexyl ether, ditridecafluorooctyl ether, and diheptadecafluorododecyl ether.

As a perfluoroalkylcarboxylic acid, for example, a carboxylic acid having 3 to 41 fluorine atoms and 1 to 21 carbon atoms can be used. Examples of the same include pentafluoroethanoic acid, pentafluoropropionic acid, heptafluoropropionic acid, heptafluorobutanoic acid, nonafluorohexanoic acid, tridecafluorooctanoic acid, heptadecafluorododecanoic acid, and metal (alkali metal, alkali earth metal, etc.) salt of the same.

As a perfluoroalkyl alcohol, for example, an alcohol having 3 to 41 fluorine atoms and 1 to 20 carbon atoms can be used. Examples of the same include pentafluoroethanol, pentafluoropropanol, heptafluoropropanol, heptafluorobutanol, nonafluorohexanol, tridecafluorooctanol, and heptadecafluorododecanol. Apart from these, adducts of the same with 1 to 20 ethylene oxides are also included in the examples.

Among the examples of the hydrophobic substance (C2) containing a hydrocarbon group having a fluorine atom, perfluoroalkyl ether, perfluoroalkyl carboxilic acid, and perfluoroalkyl alcohol are preferred with a view to improving the leakage resistance of an absorbent article to be obtained. Among these, perfluoroalkyl ether and perfluoroalkyl carboxylic acid are preferred further. Among these, perfluoroalkyl carboxylic acid is preferred particularly. Among examples of the same, pentafluoropropanoic acid is preferred most.

As a hydrophobic substance (C3) having a polysiloxane structure, a silicone, a modified silicone, or the like can be used. Examples of the same include polydimethyl siloxane, polyether-modified polysiloxane {polyoxyethylene-modified polysiloxane, poly(oxyethylene-oxypropylene)-modified polysiloxane, etc.}, carboxyl-modified polysiloxane, epoxy-modified polysiloxane, amino-modified polysiloxane, alkoxy-modified polysiloxane, and mixtures of the same.

Among the examples of hydrophobic substances (C3) having a polysiloxane structure, silicones and modified silicones are preferred with a view to improving the leakage resistance of an absorbent article to be obtained. Among these, modified silicones are preferred more, among which polyether-modified polysiloxane, amino-modified polysiloxane, and carboxyl-modified polysiloxane are preferred further more. Among these, polyether-modified polysiloxane and amino-modified polysiloxane are preferred particularly. Among these, amino-modified polysiloxane is preferred most.

In the case where a modified silicone is used, the position(s) at which an organic group(s) (modifying group (s)) is introduced is not limited particularly, and it may be at a side chain of polysiloxane, at terminals on both sides of

polysiloxane, at one terminal on one side of polysiloxane, or at both of a side chain and terminals on both sides of polysiloxane. Among these, an organic group(s) (modifying group(s)) preferably is introduced either at a side chain of polysiloxane, or at both of a side chain and terminals on both sides of polysiloxane, with a view to improving the leakage resistance of an absorbent article to be obtained. Organic groups (modifying groups) more preferably are introduced at a side chain and terminals on both sides of polysiloxane.

Examples of an organic group (modifying group) of a polyether-modified polysiloxane include groups having a polyoxyethylene group, a poly(oxyethylene-oxypropylene) group; and the like. The content (number) of oxyethylene groups and/or oxypropylene groups contained in a polyether-modified polysiloxyane preferably is 2 to 40 per one polyether-modified polysiloxane molecule, more preferably 5 to 30, particularly preferably 7 to 20, and most preferably 10 to 15. In the case where the foregoing content is in such a range, a further improved leakage resistance and the like of an absorbent article are obtained. In the case where an oxyethylene group and an oxypropylene group are contained, the content of the oxyethylene group preferably is 1 wt% to 30 wt%, more preferably 3 wt% to 25 wt%, and particularly preferably 5 wt% to 20 wt% based on the weight of polysiloxane. In the case where the content thereof is in such a range, a further improved leakage resistance and the like of an absorbent article are obtained.

Examples of an organic group (modifying group) of a carboxyl-modified polysiloxane include a group containing a carboxyl group, and the like. Examples of an organic group (modifying group) of an epoxy-modified polysiloxane include a group containing an epoxy group, and the like. Examples of an organic group (modifying group) of an amino-modified polysiloxane include a group containing an amino group (primary, secondary, or tertiary amino group), and the like.

The content of an organic group (modifying group) in such a modified silicone preferably is 400 KOHmg/g to 10000 KOHmg/g as a carboxyl equivalent, an epoxy equivalent, or an amino equivalent, more preferably 600 KOHmg/g to 5000 KOHmg/g, particularly preferably 800 KOHmg/g to 3000 KOHmg/g, and most preferably 1000 KOHmg/g to 1500 KOHmg/g. In other words, the lower limit of the content of the organic group of the modified silicone preferably is 400 KOHmg/g as a carboxyl equivalent, an epoxy equivalent, or an amino equivalent, more preferably 600 KOHmg/g, particularly preferably 800 KOHmg/g, and most preferably 1000 KOHmg/g. Likewise, the upper limit of the same preferably is 10000 KOHmg/g, more preferably 5000 KOHmg/g, particularly preferably 3000 KOHmg/g, and most preferably 1500 KOHmg/g. In the case where the limits are in the foregoing ranges, respectively, a further improved leakage resistance of an absorbent article can be obtained.

Examples of an organic group (modifying group) of an alkoxy-modified polysiloxane include alkoxy groups such as methoxy, ethoxy, butoxy, octyloxy, undodecyloxy, hexadecyloxy, and octadecyloxy. The number of carbon atoms in an alkoxy group preferably is 1 to 40, more preferably 5 to 30, particularly preferably 10 to 20, and most preferably 12 to 18. In the case where the number of the same is in such a range, a further improved leakage resistance of an absorbent article is obtained.

The hydrophobic substance (C) preferably has a viscosity at 25˚C in a range of 10 mPa·s to 2000 mPa·s, more preferably 15 mPa·s to 1500 mPa·s, and particularly preferably 20 mPa·s to 1000 mPa·s. In other words, the lower limit of the viscosity of the hydrophobic substance (C) at 25˚C preferably is 10 mPa·s, more preferably 15 mPa·s, and particularly preferably 20mPa·s. Likewise, the upper limit of the same preferably is 2000 mPa·s, more preferably 1500 mPa·s, and particularly preferably 1000 mPa·s. In the case where the viscosity is in such a range, a further improved leakage resistance of an absorbent article is obtained.

The HLB value of the hydrophobic substance (C) preferably is 1 to 10, more preferably 2 to 8, and particularly preferably 3 to 6. In other words, the lower limit of the HLB value of (C) preferably is 1, more preferably 2, and particularly preferably 3. Likewise, the upper limit of the same preferably is 10, more preferably 8, and particularly preferably 6. In the case where the HLB value thereof is in such a range, a further improved leakage resistance of an absorbent article is obtained.

The content of the hydrophobic substance (C) preferably is 0.001 wt% to 3 wt%, more preferably 0.005 wt% to 2 wt%, particularly preferably 0.07 wt% to 1.5 wt%, and most preferably 0.1 wt% to 1 wt% based the weight of the crosslinked polymer (A). In other words, the lower limit of the content of (C) preferably is 0.001 wt%, more preferably 0.005 wt%, particularly preferably 0.07 wt%, and most preferably 0.1 wt% based on the weight of (A). Likewise, the upper limit of the same preferably is 3 wt%, more preferably 2 wt%, particularly preferably 1.5 wt%, and most preferably 1 wt%. In the case where the foregoing content is in such a range, absorption amount under diffusion and water-retention amount are in more preferable ranges.

As long as the absorbent resin particle of the present invention has a structure such that a part or an entirety of the hydrophobic substance (C) is contained in the inside of each particle of the absorbent resin particle such that each particle of the absorbent resin particle contains a connection (RC) formed with the hydrophobic substance (C), and the absorbent resin particle being obtainable by either one of methods (1) and (2):

the method (1) including forming the connection (RC) and thereafter mixing the hydrophobic substance (C) with a hydrogel of the crosslinked polymer (A) or a polymerization solution of the crosslinked polymer (A); and

the method (2) including forming the connection (RC) while the crosslinked polymer (A) is being produced.

However, the structure of the absorbent resin particle containing a part or an entirety of the hydrophobic substance (C) in the inside of each particle thereof preferably is either a structure (1) such that a connection (connecting part) (RC) formed with the hydrophobic substance (C) is contained in the inside of each particle of the absorbent resin particle, or a structure (2) such that a material (D) obtained by coating or impregnating a part or an entirety of a surface of the hydrophilic material (d1) or the hydrophobic material (d2) with the hydrophobic substance (C) is contained in the inside of each particle of the absorbent resin particle.

The following describes a structure (1) such that a connection (RC) formed with the substance (C) is contained in the inside of each particle of the absorbent resin particle.

The connection (RC) refers to a sandwich structure expressed as (A)-(C)-(A), which is formed in a manner such that at least half of an entire surface of the hydrophobic substance (C) is in contact with the crosslinked polymer (A).

It should be noted that in the case where the hydrophobic substance (C) is present only on a surface of the absorbent resin particle, a two-layer structure of (A)-(C) is formed, which does not form the foregoing sandwich structure of (A)-(C)-(A). In other words, such a two-layer structure is not included in examples of the connection (RC) on the present invention.

Therefore, the structure such that the connection (RC) is contained in the inside of each particle of the absorbent resin particle refers to the structure such that the foregoing sandwich structure {(A)-(C)-(A)} is present in the inside of each particle of the absorbent resin particle.

It should be noted that preferably the hydrophobic substance (C) is present also on a part of a surface of each particle of the absorbent resin particle. More preferably, the hydrophobic substance (C) is present on a surface of each particle of the absorbent resin particle, and the substance (C) on the particle surface and the substance (C) in the inside the particle are linked continuously.

The shape of the connection (RC) is not limited particularly, and it preferably is in a layer form, a plate form, a rod form, a tubular form, a spherical form, a reticulate form, or in a combination of the same, with a view to improving the leakage resistance and the like of an absorbent article to be obtained. More preferably, it is in a layer form or a plate form. Further more preferably, it is in a layer form. Regarding the size (dimension of longest portion, unit: μm) of the connection (RC), it preferably includes a straight-line portion of 10 μm to 1000 μm, more preferably 50 μm to 800 μm, particularly preferably 100 μm to 700 μm, and most preferably 200 μm to 600 μm. With a straight-line part in such a range, a further improved leakage resistance of an absorbent article can be obtained.

In the case where the hydrophobic substance (C) is the hydrophobic substance (C2) containing a hydrocarbon group having a fluorine atom and/or the hydrophobic substance (C) having a polysiloxane structure, the shape and size of the connection (RC) can be measured by, for instance, performing the mapping of fluorine atom and/or silicon atom, etc. with use of an electron probe microanalyzer (EPMA) (for example, "JXA-862 1MX" manufactured by JEOL Ltd.). Alternatively, in the case where the hydrophobic substance (C) is the hydrophobic substance (C1) containing a hydrocarbon group, the shape and size thereof can be measured by cutting a absorbent resin particle of the present invention with use of a microtome, washing the face of section with a nonpolar solvent such as hexane and/or dimethyl ether, etc., and thereafter observing the face with use of a scanning electron microscope (SEM).

In the method (1), the connection (RC) can be formed by processing the hydrophobic substance (C) into a pulverized film form, a bead form, a rod form, or a fibrous form. The hydrophobic substance in the pulverized film form preferably has a weight-average particle diameter of 20 μm to 1000 μm, more preferably 50 μm to 500 μm, and particularly preferably 100 μm to 400 μm. The bead form preferably has a weight-average particle diameter of 0.5 μm to 100 μm, more preferably 2 μm to 300 μm, and particularly preferably 5μ m to 100 μm. The rod form preferably has a length of 20 μm to 1000 μm, more preferably 50 μm to 500, and particularly preferably 100 μm to 400 μm, and a diameter of 0.5 μm to 50 μm, more preferably 1 μm to 30 μm, and particularly preferably 2 μm to 15 μm. The fibrous form preferably has a length of 20 μm to 1000 μm, more preferably 50 μm to 500 μm, and particularly preferably 100 μm to 400 μm, and a diameter of preferably 0.5 μm to 50 μm, more preferably 1 μm to 30 μm, and particularly preferably 2 μm to 15 μm. With the dimensions in such ranges, a further improved leakage resistance of an absorbent substance can be obtained.

Examples of a connection (RC1) formed with the hydrophobic substance (C1) containing a hydrocarbon group include: bead-form materials such as polystyrene beads and polyethylene beads; and pulverized-film-form materials (weight average particle size: 20 μm to 1000 μm) such as a material obtained by pulverizing a polyethylene film (for example, "SE625M" and "UB-1" manufactured by TAMAPOLY Co., Ltd.), and a material obtained by pulverizing a polystyrene film (for example, "OPS" manufactured by Asahi Kasei Co., Ltd.). Examples of a connection (RC3) formed with the hydrophobic substance (C3) containing polysiloxane include a silicone bead material {for example, "TOSPEARL 240" manufactured by GE Toshiba Silicones (indeterminate-form silicone resin fine powder, weight-average particle diameter: 4 μm), "TOSPEARL 3120" (spherical-form silicone resin fine powder, weight-average particle diameter: 12 μm), "TOSPEARL 145" (spherical-form silicone resin fine powder, weight-average particle diameter: 4.5 μm)}. Examples

of a connection (RC2) formed with the hydrophobic substance (C2) containing a hydrocarbon group having a fluorine atom include a material obtained by pulverizing a fluorine film {for example, FLUON PTFE" (polytetrafluoroethylene film), "FLUON PFA" (film of a copolymer of tetrafluoroethylene and perfluoroethylene) or "FLUON AFLAS" (film of a copolymer of tetrafluoroethylene and propylene) manufactured by Asahi Glass Co., Ltd., etc.} (weight-average particle diameter: 20 μm to 1000μ m).

With a view to improving the leakage resistance of an absorbent article, a crushed film is preferable among the above-described materials, and a crushed fluorine film is further preferable.

The method for mixing the crosslinked polymer (A) and the connection (RC) is not limited particularly as long as the hydrophobic substance (C) is present in the inside of the crosslinked polymer (A) {in other words, the crosslinked polymer (A) and the hydrophobic substance (C) form a sandwich structure}.

However, the connection (RC) preferably is mixed, not with a dried-form crosslinked polymer (A), but with a hydrogel of the crosslinked polymer (A) or a polymerization solution of the same, and more preferably is mixed with a hydrogel-of the crosslinked polymer (A). It should be noted that in the mixing operation the materials preferably are mixed homogeneously in a kneading-like manner.

When the crosslinked polymer (A) is obtained by the aqueous solution polymerization method, the timing of mixing and kneading the connection (RC) with the crosslinked polymer (A) is not limited particularly, and the foregoing timing may be either during the polymerization process, immediately after the polymerization process, during the process of crushing (mincing) a hydrogel, during the process of drying the hydrogel, or the like. Among these, with a view to improving the leakage resistance and the like of an absorbent article, the timing preferably is immediately after the polymerization process or during the process of crushing (mincing) a hydrogel, and more preferably during the process of crushing (mincing) a hydrogel.

When the crosslinked polymer (A) is obtained by the reverse-phase suspension polymerization method or the emulsion polymerization method, the timing of mixing the connection (RC) with the crosslinked polymer (A) is not limited particularly, and the foregoing timing may be either during a polymerization process {whereby the connection (RC) is formed while the crosslinked polymer (A) is being formed}, immediately after the polymerization process, during the dehydration process (during a process of dehydrating the same until moisture becomes approximately 10 wt%}, immediately after the dehydration process, during the process of removing by distillation an organic solvent used for polymerization, during the process of drying the hydrogel, or the like. Among these, with a view to improving the leakage resistance and the like of an absorbent article, the timirig preferably is during the polymerization process, immediately after the polymerization process, during the dehydration process, immediately after the dehydration process, or during the process for removing by distillation an organic solvent used for polymerization, and further preferably, during the polymerization process, or immediately after the polymerization process.

In the case where the mixing is carried out during the process of drying the hydrogel, a conventional mixer can be used, such as bexmill, a rubber chopper, pharmamill, a mincing machine, an impact crusher, or a roll crusher as a mixing machine. In the case where the mixing is carried out in a polymerization solution, a device with a relatively high stirring power such as a homomixer or a biomixer can be used. Alternatively, in the case where the mixing is carried out during the process of drying a hydrogel, a kneading machine such as a SV mixer may be used.

In the method (2) in which the connection (RC) is formed while the crosslinked polymer (A) is being formed, the connection (RC) is formed by polymerization of the crosslinked polymer (A) in the presence of the hydrophobic substance (C). Alternatively, the connection may be formed in the following manner: the hydrophobic substance (C) is dissolved or emulsified (dispersed) in the polymerization solution for forming the crosslinked polymer (A), and the hydrophobic substance (C) is deposited in process of the polymerization for forming the crosslinked polymer (A).

The hydrophobic substance (C) may be used in a state of being dissolved and/or emulsified in water and/or a volatile solvent (however, no emulsifier is used). The volatile solvent preferably used is, from the viewpoint of removability, a volatile solvent that exhibits a vapor pressure at 20˚C in a range of 0.13 Pa to 5.3 Pa, more preferably 0.15 Pa to 4.5 Pa, and particularly preferably 0.23 Pa to 3.8 Pa.

Examples of the volatile solvent include alcohols having 1 to 3 carbon atoms (methanol, ethanol, isopropyl alcohol, etc.), hydrocarbons having 5 to 8 carbon atoms (pentane, hexane, cyclohexane, toluene, etc.), ethers having 2 to 4 carbon atoms (dimethylether, diethylether, tetrahydrofuran, etc.), ketones having 3 to 4 carbon atoms (acetone, methylethylketone, etc.), and esters having 3 to 5 carbon atoms (ethyl formate, ethyl acetate, isopropyl acetate, diethyl carbonate, etc.). In the case where water and/or a volatile solvent are used, the amount of the same used preferably is 1 wt% to 900 wt%, more preferably 5 wt% to 700 wt%, and particularly preferably 10 wt% to 400 wt% based on the weight of the hydrophobic substance (C). In the case where water and a volatile solvent are used, the amount of water used preferably is 50 wt% to 98 wt%, more preferably 60 wt% to 95 wt%, and particularly preferably 70 wt% to 90 wt% based on the weight of water and the volatile solvent.

The following describes a structure (2) such that a material (D) obtained by coating or impregnating a part or an entirety of a surface of the hydrophilic material (d1) or the hydrophobic material (d2) with the hydrophobic substance (C) is contained in the inside of each particle of the absorbent resin particle.

As the material (D), a hydrophilic material (d1) or a hydrophobic material (d2) coated or impregnated with the hydrophobic substance (C), or the like, can be used.
Examples of the hydrophilic material (d1) include hydrophilic organic polymers (d11) and hydrophilic inorganic substances (d12).
Examples of the hydrophilic organic polymer (d11) include a polymer electrolyte {crosslinked polymer (A), etc.}, a cellulose (pulp, cotton, sawdust, straw, etc.), wool, microfibril, and bacteria cellulose.
Examples of the hydrophilic inorganic substance (d12) include glass, silica gel, silica, and clay.
Examples of the hydrophobic material (d2) include hydrophobic organic polymers (d21) and hydrophobic inorganic substances (d22).
As the hydrophobic organic polymer (d21), a synthetic resin having a weight average molecular weight in a range of 1000 to 1000000 can be used. Examples of the same include the hydrophobic substance (C1) applicable for forming the connection (RC), polystyrene resins, polyethylene resins, polypropylene resins, acrylic resins, polyester resins, polyamide resins, epoxy resins, and urethane resins.
Examples of the polystyrene resin include polystyrene, styrene-ethylene copoymer, and styrene butadiene copolymer.
Examples of the polyethylene resin include high-density polyethylene and low-density polyethylene.
Examples of the polypropylene resin include polypropylene and etylene-propylene copolymer.
Examples of the acrylic resin include methyl (meth)acrylates, ethyl (meth)acrylates, and polymers of (meth) acryl ester of long-chain alkyl alcohol having 8 to 22 carbon atoms.
Examples of the polyester resin include polyethylene terephthalate and polybutadiene terephthalate.
Examples of the polyamide resin include nylon.
Examples of the epoxy resin include materials obtained by curing the conventional epoxy resins.
Examples of the urethane resin include materials obtained by curing conventional isocyanates and polyols.
Examples of the hydrophobic inorganic substance (d22) include carbon fiber, kaolin, talc, mica, bentonite, sericite, asbestos, and Shirasu (volcanic ash).
Among these, the hydrophilic materials (d1) are preferred. Among these, the hydrophilic organic polymers (d11) are preferred further, and among these, cellulose and polymer electrolyte are preferred particularly.
Regarding the shape of the hydrophilic material (d1) and the hydrophobic material (d2), the shape may be any one of an indeterminate shape (crushed shape), a spherical shape, a film shape, a rod shape, a fibrous shape, and the like, among which the indeterminate shape (crushed shape) and the film shape are preferred, among which the indeterminate shape (crushed shape) is preferred further.
The hydrophilic material (d1) and the hydrophobic material (d2) preferably have a volume-average particle diameter of 1 $\mu$m to 1000 $\mu$m, more preferably 5 $\mu$m to 500 $\mu$m, particularly preferably 10 $\mu$m to 250 $\mu$m, further particularly preferably 15 $\mu$m to 150 $\mu$m, and most preferably 20 $\mu$m to 100 $\mu$m. In such a range, a further improved leakage resistance of an absorbent article is obtained.
It should be noted that the volume-average particle diameter is measured by the light scattering method (solvent: methanol) according to JIS Z8825-1:2001.
In the case where the material (D) is the material obtained by coating or impregnating the hydrophobic material (d2) with the hydrophobic substance (C), among the examples of the hydrophobic substance (C1) containing a hydrocarbon group, the following is used as the hydrophobic material (C) used for coating or impregnation, with a view to improving the leakage resistance and the like of an absorbent article: preferably a polyolefin resin, a wax, or a long-chain fatty acid ester; more preferably a wax or a long-chain fatty acid ester; particularly preferably a wax; and most preferably paraffin wax. Likewise, among examples of the hydrophobic substance (C2) containing a hydrocarbon group having a fluorine atom, the following is used as the hydrophobic material (C): preferably a perfluoroalkane or a perfluoroalkyl ether; more preferably perfluoroalkyl ether; particularly preferably dipentafluoropropyl ether, diheptafluoropropyl ether, diheptafluorobutyl ether, or dinonafluorohexyl ether; and most preferably diheptafluoropropyl ether, or diheptafluorobutyl ether. Further likewise, among examples of the hydrophobic substance (C3) having a polysiloxane structure, the following is used as the hydrophobic material (C): preferably polydimethyl siloxane, or alkoxy-modified polysiloxane; and further preferably polydimethyl siloxane.
Among the above-mentioned hydrophobic substances, the hydrophobic substances (C3) having a polysiloxane structure are preferred.
In the case where the material (D) is a material obtained by coating or impregnating the hydrophilic material (d1) with the hydrophobic substance (C), among the examples of the hydrophobic substance (C1) containing a hydrocarbon group, the following is used as the hydrophobic material (C) used for coating or impregnation, with a view to improving the leakage resistance and the like of an absorbent article: preferably a polyolefin resin derivative, a wax derivative, or an ester of a long-chain fatty acid; more preferably a wax derivative, or an ester of a long-chain fatty acid; particularly preferably an ester of a long-chain fatty acid; most preferably pentaerythritol monooleate, or sorbitol monooleate. Likewise, among the examples of the hydrophobic substance (C2) containing a hydrocarbon group having a

fluorine atom, the following is used as the hydrophobic material (C) used for coating or impregnation: preferably a perfluoroalkyl carboxylic acid, or a perfluoroalkyl alcohol; more preferably a metal salt of a perfluoroalkyl carboxylic acid, or a perfluoroalkyl alcohol; particularly preferably a metal salt of a perfluoroalkyl carboxylic acid. Further likewise, among the examples of the hydrophobic substance (C3) having a polysiloxane structure, the following is used as the hydrophobic material (C) used for coating or impregnation: preferably a polyether-modified polysiloxane, a carboxyl-modified polysiloxane, an epoxy-modified polysiloxane, or an amino-modified polysiloxane; more preferably carboxyl-modified polysiloxane, an epoxy-modified polysiloxane, or an amino-modified polysiloxane; particularly preferably a carboxyl-modified polysiloxane or an amino-modified polysiloxane; most preferably an amino-modified polysiloxane.

Among the above-mentioned hydrophobic substances, the hydrophobic substances (C3) having a polysiloxane structure are preferred.

The content of the hydrophobic substance (C) in the material (D) is, based on the weight of the material (D), preferably in a range of 0.001 wt% to 3 wt%, more preferably 0.005 wt% to 1 wt%, particularly preferably 0.07 wt% to 0.5 wt%, and most preferably 0.1 wt% to 0.3 wt%. In such a range, a further improved leakage resistance of an absorbent article is obtained.

The content of the material (D) is, based on the weight of the crosslinked polymer (A), preferably in a range of 0.1 wt% to 50 wt%, more preferably 0.5 wt% to 20 wt%, particularly preferably 1 wt% to 10 wt%, and most preferably 2 wt% to 4 wt%. In such a range, a further improved leakage resistance of an absorbent article is obtained.

In the case where the absorbent resin particle has a structure containing the material (D), the absorbent resin particle can be produced by the method (1) of mixing and kneading the material (D) and the crosslinked polymer (A), or the method (2) of causing polymerization for forming the crosslinked polymer (A) in the presence of the material (D).

The mixing method (1) for mixing the crosslinked polymer (A) and the material (D) is not limited particularly as long as the material (D) and the crosslinked polymer (A) are mixed so that the material (D) is present in the inside of the crosslinked polymer (A) {preferably the crosslinked polymer (A) and the material (D) form a sandwich structure}.

However, the material (D) preferably is mixed, not with a dried-form crosslinked polymer (A), but with a hydrogel of the crosslinked polymer (A) or a polymerization solution of the same, and more preferably is mixed with a hydrogel of the crosslinked polymer (A). It should be noted that in the mixing operation the materials preferably are mixed homogeneously in a kneading-like manner.

The timing of mixing the material (D) with the crosslinked polymer (A) is not limited particularly, and is identical to the timing of mixing the connection (RC).

Regarding the device used for the foregoing mixing/kneading process, the same device used for mixing the connection (RC) can be used.

In the method (2) of causing polymerization for forming the crosslinked polymer (A) in the presence of the material (D), the polymerization may be caused in a state in which the material (D) is emulsified or dispersed in the polymerization solution for forming the crosslinked polymer (A).

When a part or an entirety of a surface of the hydrophilic material (d1) or the hydrophobic material (d2) is coated or impregnated with the hydrophobic substance (C), the hydrophobic substance (C) may be dissolved or emulsified/dispersed in a solvent, or alternatively, heated to a temperature above its melting point so as to become molten. By so doing, the hydrophobic substance (C) can be used in a liquid state.

The coating or impregnation of the hydrophilic material (d1) or the hydrophobic material (d2) with the hydrophobic substance (C) can be achieved by spraying the above-described liquid over the hydrophilic material (d1) or the hydrophobic material (d2), or dipping the hydrophilic material (d1) or the hydrophobic material (d2) in the above-described liquid. It should be noted that it is possible that the coating or impregnation can be achieved by bringing either the hydrophobic substance (C) in a solid state or in an emulsion dispersion of the hydrophobic substance (C) into contact with the the hydrophilic material (d1) or the hydrophobic material (d2) and subsequently heating the same at or above the melting point of the substance (C).

In the case where a solvent is used for coating or impregnation, the solvent preferably is removed by distillation. In this case, the content of the organic solvent after the removal by distillation preferably is 10 wt% to 0.01 wt%, more preferably 5 wt% to 0.05 wt%, particularly preferably 3 wt% to 0.1 wt%, and most preferably 1 wt% to 0.5 wt% based on the weight of the material (D). With the content in such a range, a further improved leakage resistance (particularly water-retention amount) of an absorbent article is obtained. In the case where water is contained in the solvent, the content of moisture after the distillation preferably is 0 wt% to 20 wt%, more preferably 0 wt% to 10 wt%, particularly preferably 0 wt% to 5 wt%, and most preferably 0 wt% to 2 wt% based on the weight of the material (D). With the content thereof in such a range, a further improved leakage resistance (particularly water-retention amount) of an absorbent article is obtained.

It should be noted that the method for removing the solvent and the method for determining the content of the solvent are identical to those in the case of the crosslinked polymer (A).

The solvent that can be used is not limited particularly as long as it dissolves the hydrophilic substance (C), and the above-described water and/or volatile solvent are preferred.

Examples of a mixer applicable for spraying, dipping, or bringing into contact include a Nauta-mixer and a turbulizer.

In the case of the hydrogel particle of an absorbent resin particle containing water, the hydrogel may be crushed as required. The size (maximum diameter) of the hydrogel particle obtained by crushing preferably is 50 μm to 10 cm, more preferably 100 μm to 2 cm, and particularly preferably 1 mm to 1 cm. With the size in such a range, improved dryness is achieved in the drying process.

The crushing can be carried out by the same method as that for the crosslinked polymer (A).

In the case where a solvent (containing a volatile solvent) is used in the production of the absorbent resin particle, the solvent can be removed by distillation after polymerization.

In the case where the solvent contains an organic solvent, the content of the organic solvent after the removal by distillation preferably is 10 wt% to 0.01 wt%, more preferably 5 wt% to 0.05 wt%, particularly preferably 3 wt% to 0.1 wt%, and most preferably 1 wt% to 0.5 wt% based on the weight of the absorbent resin particle. With the content in such a range, a further improved absorption performance (particularly water-retention amount) of the absorbent resin particle is obtained.

In the case where water is contained in the solvent, the content of moisture after the distillation preferably is 0 wt% to 20 wt%, more preferably 0 wt% to 10 wt%, particularly preferably 0 wt% to 5 wt%, and most preferably 0 wt% to 2 wt% based on the weight of the absorbent resin particle. With the content thereof in such a range, further improved absorption performance (particularly water-retention amount) and handling property after drying (powder fluidity of the absorbent resin particle, etc.) are obtained.

The method for determining the content of an organic solvent and water and the method for removing a solvent by distillation are identical to those in the case of the crosslinked polymer (A).

The absorbent resin particle may be pulverized. In the case where the absorbent resin particle contains a solvent, it is preferable to pulverize the same after removing the solvent by distillation (drying).

In the case where it is pulverized, the weight-average particle diameter of the pulverized absorbent resin particle preferably is 100 μm to 800 μm, more preferably 200 μm to 500 μm, and particularly preferably 300 μm to 400 μm. With the diameter in such a range, a further improved handling property after pulverizing (powder fluidity of the absorbent resin particle, etc.) is obtained. It should be noted that the weight-average particle diameter can be determined in the same manner as that in the case of the crosslinked polymer (A).

The smaller the content of fine particles is, the better the absorption performance is. Preferably, the content of fine particles having the particle diameter of 106 μm or less is 3 wt% or less with respect to the total weight of the particles, and more preferably, the content of fine particles having the particle diameter of 150 μm or less is 3 wt% or less with respect to the total weight of the particles. The content of the fine particles can be determined by using a plot generated when the above-mentioned weight-average particle diameter is determined.

For the pulverization and the particle size adjustment, the same methods as those in the case of the crosslinked polymer (A) can be used.

The shape of particles of the absorbent resin particle is not particularly limited. Examples of the shape include an indeterminate crushed shape, a scale shape, a pearl shape, and a rice grain shape. Among these shapes, the indeterminate crushed shape is preferable because the particles in such a shape easily can be entangled with fibrous materials when used for a disposable diaper, etc., and there is little possibility of the crosslinked polymer particles falling off from the fibrous materials.

The absorbent resin particle may be subjected to surface crosslinking as required. As a crosslinking agent for carrying out the surface crosslinking (surface crosslinking agent), the same ones as the internal crosslinking agents (b) may be used. As the surface crosslinking agent, with a view to improving the absorption performance and the like of the absorbent resin particle, a crosslinking agent (b3) having at least two functional groups capable of reacting with a water-soluble substituent of the water-soluble vinyl monomer (a1) and/or a water-soluble substituent generated by hydrolysis of the vinyl monomer (a2) is used preferably. Among examples of the same, polyglycidyls are preferred further, among which ethylene glycol diglycidyl ether, and glycerin diglycidyl ether are preferred particularly. Ethylene glycol diglycidyl ether is preferred most.

In the case where the surface crosslinking is performed, the content of the surface crosslinking agent preferably is 0.001 wt% to 7 wt%, more preferably 0.002 wt% to 5 wt%, and particularly preferably 0.003 wt% to 4 wt% based on a total amount of the vinyl monomers (a1) and/or (a2), the internal crosslinking agent (b), and the other vinyl monomer (a3) used if necessary. In other words, in this case, the upper limit of the content of the surface crosslinking agent preferably is 7 wt%, more preferably 5 wt%, and particularly preferably 4 wt% based on the total weight of (a1) and/or (a2), (b), and (a3). Likewise, the lower limit of the content of the surface crosslinking agent preferably is 0.001 wt%, more preferably 0.002 wt%, and particularly preferably 0.003 wt%. With the content of the same in such a range, a further improved absorption performance is obtained. The surface crosslinking is achieved by, for example, spraying an aqueous solution containing a surface linking agent over the absorbent resin particle or impregnating the absorbent resin particle with the aqueous solution, and subsequently heating the same (100˚C to 200˚C).

The absorbent resin particle of the present invention preferably further contains a diffusing-penetrating agent (E) as a constituent component. By containing a diffusing-penetrating agent (E), a further improved absorption performance (absorption amount and absorption rate) can be obtained.

The diffusing-penetrating agent (E) is an additive for improving the diffusing and penetrating property of a liquid in the inside of each particle of the absorbent resin particle, and as the diffusing-penetrating agent (E), a conventionally known surface active agent or the like can be used (for example, surface active agents described in "Suiyosei Kobunshi no Saishin Gijutsu (Latest Technology of Water-Soluble Polymer)' published by CMC, May 2000 and "Kaimen Kasseizai no Oyo Gijutsu (Applications Technology of SurfaceActive Agents)' published by CMC, December 2002). In the case where the crosslinked polymer (A) is obtained by the aqueous solution polymerization, among the surface active agents, the following can be used: anionic surface active agents; non-ionic surface active agents; cationic surface active agents; and amphoteric surface active agents. Such a surface active agent may be used singly, or two or more kinds of the same may be used in combination.

Examples of the anionic surface active agent include:

ether carboxylic acid having 12 to 300 carbon atoms and salt thereof [sodium polyoxyethylene (polymerization degree: 1 to 5) lauryl ether acetate, disodium polyoxyethylene (polymerization degree:1 to 5) lauryl sulfosuccinate, etc.];
alkyl (ether) sulfuric ester salt having 12 to 300 carbon atoms [sodium lauryl sulfate, sodium polyoxyethylene (polymerization degree: 1 to 5) lauryl sulfate, triethanolamine polyoxyethylene (polymerization degree: 1 to 5) lauryl sulfate, polyoxyethylene (polymerization degree: 1 to 5) coconut oil fatty acid monoethanol amide sodium sulfate, etc.]
alkyl (or alkyl phenyl) sulfonic acid salt having 12 to 24 carbon atoms [sodium dodecylbenzene sulfonate, sodium diethyl hexyl sulfosuccinate, etc.];
alkyl (ether) phosphate ester salt having 12 to 300 carbon atoms [sodium lauryl phosphate, sodium polyoxyethylene (polymerization degree: 1 to 100) lauryl ether phosphate, etc.];
fatty acid salt having 12 to 24 carbon atoms [sodium laurate, triethanolamine laurate, etc.];
acylated amino acid salt [sodium coconut oil fatty acid methyl taurine, sodium coconut oil fatty acid sarcosine, triethanolamine coconut oil fatty acid sarcosine, triethanolamine N-coconut oil fatty acid acyl-L glutamate, sodium N-coconut oil fatty acid acyl-L glutamate, sodium lauroylmethyl-β-alanine, etc.]; and
others [disodium polyoxyethylene (polymerization degree: 1 to 5) lauroyl ethanol amide sulfosuccinate, etc.], and the like.

Furthermore, examples of the nonionic surface active agent include:

aliphatic alcohol (the number of carbon atoms: 12 to 24) alkylene oxide (the number of carbon atoms: 2 to 5) adduct (polymerization degree: 1 to 5) [lauryl alcohol ethylene oxide adduct (polymerization degree: 3), oleyl alcohol ethylene oxide adduct (polymerization degree: 3), cachalot alcohol ethylene oxide adduct (polymerization degree: 3), etc.];
polyoxy alkylene (the number of carbon atoms: 2 to 8, polymerization degree: 1 to 5) higher fatty acid (the number of carbon atoms: 12 to 24) ester [polyethylene glycol (polymerization degree: 3) monostearate, polyethylene glycol (polymerization degree: 3) distearate, etc.];
polyvalent (the number of valences: 2 to 10) alcohol fatty acid (the number of carbon atoms: 8 to 24) ester [glycerine monostearate, ethyleneglycol monostearate, sorbitan lauric acid (mono/di) ester, sorbitan palmitic acid (mono/di) ester, sorbitan stearic acid (mono/di) ester, sorbitan oleic acid (mono/di) ester, sorbitan coconut oil (mono/di) ester, etc.];
polyoxyalkylene (the number of carbon atoms: 2 to 8, polymerization degree: 1 to 5) polyvalent (the number of valences: 2 to 10) alcohol higher fatty acid (the number of carbon atoms: 8 to 24) ester [polyoxyethylene (polymerization degree: 2) sorbitan lauric acid (mono/di) ester, polyoxyethylene (polymerization degree: 2) sorbitan palmitic acid (mono/di) ester, polyoxyethylene (polymerization degree: 3) sorbitan stearic acid (mono/di) ester, polyoxyethylene (polymerization degree: 3) sorbitan oleic acid (mono/di) ester, polyoxyethylene (polymerization degree: 2) lauric acid (mono/di) ester, polyoxyethylene (polymerization degree: 3) stearic acid (mono/di) ester, polyoxyethylene (polymerization degree: 3) oleic acid (mono/di) ester, dioleic acid methyl glucoside, etc.];
fatty acid alkanol amide [1:1 type coconut oil fatty acid diethanol amide, 1:1 type lauric acid diethanol amide, etc.];
polyoxy alkylene (the number of carbon atoms: 2 to 8, polymerization degree: 1 to 5) alkyl (the number of carbon atoms: 1 to 22) phenyl ether (polyoxyethylene (polymerization degree: 2) nonyl phenyl ether etc.), polyoxy alkylene (the number of carbon atoms: 2 to 8, polymerization degree: 1 to 5) alkyl (the number of carbon atoms: 8 to 24) amino ether, alkyl (the number of carbon atoms: 8 to 24) dialkyl (the number of carbon atoms: 1 to 6) amine oxide [lauryl dimethylamine oxide, etc.], and the like.
It should be noted that in the above, "... (mono/di) ester" means "...monoester" or "...diester". The same applies hereinafter.

Furthermore, examples of the cationic surface active agent include:

quarternary ammonium salt [stearyl trimethylammonium chloride, behenyl trimethylammonium chloride, distearyl dimethylammonium chloride, ethyl sulfuric acid lanolin fatty acid amino propylethyl dimethylammonium, etc.]; and amine salt [diethyl aminoethyl stearate amide lactate, dilauryl amine hydrochloride, oleylamine lactate, etc.] and the like.

Furthermore, examples of the amphoteric surface active agent include:

betaine type amphoteric surface active agent [coconut oil fatty acid amidepropyl dimethyl aminoacetic acid betaine, lauryl dimethyl aminoacetic acid betaine, 2-alkyl N-carboxy methyl-N-hydroxyethyl imidazolinium betaine, lauryl hydroxysulfo betaine, lauroyl amideethyl hydroxyethyl carboxymethyl betaine hydroxypropyl sodium phosphorate, etc.];

amino acid type amphoteric surface active agent [sodium β-lauryl aminopropionate, etc.] and the like.

Among the foregoing diffusing-penetrating agents (E), the anionic surface active agents and the non-ionic surface active agents are preferred with a view to improving the leakage resistance and the like of an absorbent article. Among these, alkyl (ether) sulfuric ester salt, alkyl (or alkyl phenyl) sulfonic acid salt, alkyl (ether) phosphoric ester salt, fatty acid salt, and polyvalent alcohol fatty acid ester are preferred further. Among these, sodium lauryl sulfate, sodium lauryl phosphate, sodium dodecylbenzene sulfonate, sodium diethyl hexyl sulfosuccinate, sodium lauryl phosphate, sodium laurate, sorbitan lauric acid (mono/di) ester, sorbitan palmitic acid (mono/di) ester, sorbitan stearic acid (mono/di) ester, sorbitan oleic acid (mono/di) ester, sorbitan coconut oil (mono/di) ester, and sorbitan lauric acid (mono/di) ester are preferred particularly. Among these, sodium dodecylbenzene sulfonate, and sodium diethyl hexyl sulfosuccinate are preferred most.

It should be noted that in the case where the crosslinked polymer (A) is obtained by the emulsification polymerization or the reverse-phase suspension polymerization, it is not preferable to use, as the diffusing-penetrating agent (E), the same surface active agent as the surface active agent (emulsifying-dispersing agent) used in the emulsification polymerization or the reverse-phase suspension polymerization. This is because it is considered that the use of the same surface active agent as the diffusing-penetrating agent (E) hardly improves the diffusion and penetration properties of a liquid in the inside of the absorbent resin particle. In this case, an anionic surface active agent or a cationic surface active agent different from the surface active agent used in the emulsification polymerization or the reversed-phase suspension polymerization is used preferably, and more preferably, an anionic surface active agent different from the same. As the anionic surface active agent different from the same, any one of those mentioned regarding the aqueous solution polymerization is used preferably.

Among the examples of the diffusing-penetrating agents (E), an agent having a HLB value with a greater difference from a HLB value of the hydrophobic substance (C) is preferred. The difference between the HLB values of the substance (C) and the agent (E) preferably is 1 to 10, more preferably 2 to 8, and particularly preferably 3 to 7. With the difference in such a range, a further improved leakage resistance of an absorbent article can be obtained.

The HLB value of the diffusing-penetrating agent (E) preferably is 5 to 12, more preferably 6 to 11, particularly preferably 7 to 10, and most preferably 8 to 9. With the HLB value in such a range, a further improved leakage resistance of an absorbent article can be obtained.

In the case where the diffusing-penetrating agent (E) is contained, the content of the diffusing-penetrating agent (E) preferably is 0.001 wt% to 3 wt%, more preferably 0.005 wt% to 1 wt%, particularly preferably 0.07 wt% to 0.5 wt%, and most preferably 0.1 wt% to 0.3 wt% based on the weight of the crosslinked polymer (A). With the content in such a range, a further improved leakage resistance of an absorbent article can be obtained.

In the case where the diffusing-penetrating agent (E) is contained, the agent (E) preferably is present in the inside of each particle of the crosslinked polymer (A), and more preferably on a surface of the connection (RC) or the material (D).

In the case of the aqueous solution polymerization method, the timing of causing the diffusing-penetrating agent (E) to be contained in the absorbent resin particle is not limited particularly, and the foregoing timing may be either during the polymerization process, immediately after the polymerization process, during the process of crushing (mincing) a hydrogel, during the process of drying the hydrogel, or the like. Among these, with a view to improving the leakage resistance and the like of an absorbent article, the timing preferably is immediately after the polymerization process or during the process of crushing (mincing) a hydrogel, and more preferably immediately after the polymerization process.

In the case of the reversed-phase suspension polymerization method or the emulsion polymerization method, the timing of causing the diffusing-penetrating agent (E) to be contained in the absorbent resin particle may be either immediately after the polymerization process, during a dehydration process (during a process of dehydrating the same until moisture becomes approximately 10 wt%), immediately after the dehydration process, during a process of removing

by distillation an organic solvent used for polymerization, during a process of drying the hydrogel, or the like. Among these, with a view to improving the leakage resistance of an absorbent article and the stability during polymerization, the timing preferably is immediately after the polymerization process, during the dehydration process, immediately after the dehydration process, or during the process for removing by distillation an organic solvent used for polymerization, and further preferably, immediately after the polymerization process, or during the dehydration process.

As a method for causing the diffusing-penetrating agent (E) to be contained in the absorbent resin particle and a device used for this purpose, the same method and device used for mixing the connection (RC) or the material (D) with the crosslinked polymer (A), or the like, can be used.

In order to make the diffusing-penetrating agent (E) present on a surface of the connection (RC) or the material (D), a method of first causing the agent (E) to be contained in the polymer (A) and subsequently mixing the connection (RC) {or the hydrophobic substance (C)} or the material (D) therein, or the like, can be used.

The diffusing-penetrating agent (E) may be used in a state of being dissolved and/or emulsified in water and/or a volatile solvent. As the volatile solvent, the same one as that of the case of hydrophobic substance (C) can be used, and the preferable ranges and amount to be used are identical, too.

A diffusion absorption amount of the absorbent resin particle of the present invention preferably is 40 ml to 70 ml, more preferably 45 ml to 65 ml, and particularly preferably 50 ml to 60 ml. With the absorption amount in such a range, when the absorbent resin particle of the present invention is employed in an absorbent article, a further improved leakage resistance is exhibited by the absorbent article.

The diffusion absorption amount (ml) of the absorbent resin particle is a value determined by the following measuring method.

Measuring method:

In a filtering cylindrical container (2) composed of a cylinder standing vertically (internal diameter: 60 mm, length: 50 mm) and a wire mesh (1) (mesh opening: 150 μm) at the bottom of the cylinder, 1.25 g of a sample (3) is placed so that the sample has a uniform thickness on the mesh (1). A charging tube-equipped disk (5) (diameter: 59 mm, thickness: 4 mm, weight: 29g) to which a charging tube (4) (internal diameter: 4 mm, length: 50 mm) is attached vertically with respect to a top face of the disk in a manner such that an internal vent of the tube goes through the disk to a bottom face of the disk is placed on the sample (3) in a manner such that the bottom face of the disk (5) and the sample (3) are in contact with each other. Further, a ring-shaped weight (6) (external diameter: 50 mm, diameter of internal hole: 16 mm, weight 150 g) is placed on the top face of the disk (5), and physiological saline (25°C) is poured continuously into the charging tube (4) at a rate of 10 ml/min. A time (T) from the start of the pouring of the physiological saline till the start of leakage of the physiological saline out of the mesh (1) is determined, and the diffusion absorption amount is calculated by formula (1). It should be noted that in the case where physiological saline reaches a line marked at a position 5 mm below an upper end of the charging tube (4) before leaking out of the mesh (1), the measurement is stopped when this occurs, and it is recorded that the measurement is impracticable.

$$\text{Diffusion Absorption amount (ml)} = 10\ (\text{ml/min}) \times T\ (\text{min}) \quad \ldots (1)$$

The following describes the measurement of the diffusion absorption amount in detail. FIGS. 1 and 2 schematically illustrate a device used for the measurement of the diffusion absorption amount, which are a front cross-sectional view and a perspective transparent view of the device, respectively.

The device used for measuring the diffusion absorption amount is composed of the filtering cylindrical container (2), the charging tube-equipped disk (5), and the ring-shaped weight (6) (see FIGS. 1 and 2). Further, a syringe, a beaker, a metering pump, or the like for pouring physiological saline at a uniform rate is necessary, and from the viewpoint of accuracy, a metering pump is preferable.

As long as the cylinder composing the filtering cylindrical container (2) has an internal diameter of 60 mm and a length of 50 mm, a material forming the same, etc., is not limited particularly, but a transparent material (for example, polyvinyl chloride, polycarbonate, polypropylene, or polyethylene) is preferred so as to allow the inside to be observed. The bottom of the cylinder is covered with the wire mesh (1) with an opening of 150 μm, through which physiological saline passes but the sample (3) cannot. It should be noted that the mesh is according to JIS Z8801-1:2000.

The disk composing the disk (5) equipped with the charging tube (4) has a diameter of 59 mm, a thickness of 4 mm, and a weight of 29 g (including the weight of the charging tube (4)), and preferably is made of a transparent material so as to allow the inside thereof to be observed. At the central part of the disk, the charging tube (4) with an

internal diameter of 4 mm and a length of 50 mm is placed vertically with respect to the top face of the disk, and the internal vent of the charging tube (4) goes through the disk to the bottom face of the disk, so that physiological saline poured into the charging tube (4) is poured into the sample under the disk (5). The charging tube-equipped disk (5) is inserted in the filtering cylindrical container (2) so that the sample (3) is retained between the bottom face of the disk and an upper face of the mesh (1).

The ring-shaped weight (6) has an external diameter of 50 mm, an internal hole diameter of 16 mm, and a weight of 150 g, and is configured so that the charging tube (4) of the charging tube-equipped disk (5) goes through the internal hole of the ring shape (doughnut shape) and a load is imposed on the top face of the disk. The ring-shaped weight (6) preferably is made of a metal material (steel, copper, stainless steel, lead, etc.), from a viewpoint of space.

As a syringe and a beaker, conventional instruments normally used in chemical experiments can be used, and as a metering pump, a gear pump, a tube pump, an injector pump, or the like can be used.

The measurement is carried out in the following manner. First, the filtering cylindrical container (2) is fixed so that its axial direction is directed in a vertical direction (gravity direction), and 1.25 g of the sample (3) is placed on the mesh (1) so as to have a uniform thickness (distribution). On the sample (3), the charging tube-equipped disk (5) is placed so that the bottom face of the disk is in contact with the sample (3). Further, the ring-shaped weight (6) is placed on the disk top face of the disk (5).

Subsequently, physiological saline is poured in the charging tube (4) at a rate of 10 ml/min continuously. Here, the physiological saline poured in the charging tube (4) should be free from pressure other than the atmospheric pressure and-the pressure of a column of itself in the tube. In the case where a metering pump or a syringe is used, an external diameter of an injection nozzle thereof to the charging tube (4) preferably is approximately 3 mm or less so that no jetting pressure of the metering pump or the like is applied to the physiological saline.

The time (T) from the start of the pouring of physiological saline till the start of leakage of the same out of the mesh (1) is determined, and a diffusion absorption amount is calculated from the formula (1). It should be noted that in the case where physiological saline remains in the charging tube (4) and reaches a line marked at a position 5 mm below an upper end of the charging tube (4) before leaking out of the mesh (1), the measurement is stopped when this occurs, and it is recorded that the measurement of the diffusion absorption amount is impracticable.

The temperature of physiological saline used and ambient atmosphere for the measurement is set to 25±2°C.

$$\text{Diffusion absorption amount (ml)} = 10\ (\text{ml/min}) \times T\ (\text{min}) \quad \ldots (1)$$

The absorbent resin particle of the present invention preferably exhibits an absorption time (Z) (unit: minute) of 0.5 min to 3.5 min, more preferably 0.6 min to 3 min, further more preferably 0.7 min to 2.5 min, particularly preferably 0.8 min to 2 min, and most preferably 0.9 min to 1.8 min.

The absorbent resin particle of the present invention preferably satisfies formulae (2) and (3) shown below:

$$30 \leq (X) \leq 70 \quad \ldots (2)$$

$$(Z) \leq -0.0071(Y)+2.7 \quad \ldots (3)$$

The water-retention amount (X) (g/g) refers to an amount of water retained by the absorbent resin particle that has been immersed in physiological saline for one hour, and it is measured by the following method.

<Method for measuring the water-retention amount (X)>

1.00 g of a sample is put in a sack like a teabag made of a 250-mesh nylon net (20 cm in length and 10 cm in width), and it is immersed in 1000 cc of physiological saline (salt concentration: 0.9 wt%) for one hour without stirring. Thereafter, it is hung for 15 minutes for drain. Then, the sample together with the sack is put in a centrifuge for centrifugal drying with 150 G for 90 seconds so that extra physiological saline is removed. Then, a weight (h1) including the weight of the sack is measured, and a water-retension amount (X) is calculated from the formula shown below. It should be

noted that the temperature of physiological saline used and ambient atmosphere for the measurement is set to 25±2°C.

$$(X) = \{(h1)-(h2)-1.00\}/1.00$$

(h2) represents a weight of the sack in a state of containing no sample, which is measured in the same manner as described above.

The water-retention amount (X) preferably satisfies formula (8) in place of formula (2), more preferably satisfies formula (9), particularly preferably satisfies formula (10), and most preferably satisfies formula (11). If such a formula is satisfied, an absorbent article that exhibits a further improved absorption performance under any condition and hardly causes leakage is produced readily.

$$10 \leq (X) \leq 65 \quad \ldots (8)$$

$$22 \leq (X) \leq 60 \quad \ldots (9)$$

$$25 \leq (X) \leq 55 \quad \ldots (10)$$

$$30 \leq (X) \leq 50 \quad \ldots (11)$$

The liquid permeation rate (Y) is a liquid permeation rate (ml/min) under loading of 2.14kPa of physiological saline permeating an absorbent resin particle that has been immersed in physiological saline for one hour, and is measured by the following method.

<Method for measuring liquid permeation rate (Y)>

0.32g of a sample to be measured is immersed in 50 ml of physiological saline for one hour so as to prepare a hydrogel particle.

On the other hand, a filter (mesh opening: 10 μm to 15 μm)-enclosed-type chromatograph tube (diameter (internal diameter): 25.4 mm, and length: 35 cm) equipped with a cock (internal diameter: 5 mm, length: 10 cm) and a capacity scale is fixed vertically (in gravity direction) with the cock being closed and the cock side facing downward.

Then, the above-mentioned hydrogel particle together with a physiological saline are transferred to the chromatograph tube; a pressure shaft (weight: 15.5 g, and length: 31.5 cm) equipped with a round mesh (diameter: 25 mm) with 150 μm openings (JIS Z8801-1:2000) perpendicularly at one of ends of the shaft is placed in a way in which the mesh side is a hydrogel particle side; and furthermore a weight (91.5 g) is placed thereon, and the hydrogel and physiological saline are left to stand for one minute.

The cock at the lower part of the chromatograph tube is opened, the period of time (T1: second) from when a liquid surface inside the tube indicates a marking of 60 ml till it indicates a marking of 40 ml is measured and the liquid permeation rate (Y, ml/min) is calculated from the following formula. It should be noted that temperature of physiological saline used and ambient atmosphere for the measurement is set to 25±2°C.

$$(Y) = 20\ (ml) \times 60\ /\ (T1-T2)$$

(T2) is a period of time calculated by the same operation as described above in the case where no sample to be measured is in the chromatograph tube. That is to say, it means the period of time (T2; second) necessary for a liquid amount inside the tube to decrease from 60 ml to 40 ml in the case where 50 ml of physiological saline is placed in a

filter-enclosed-type chromatograph tube equipped with a cock and a capacity scale.

An absorption time (Z) is an absorption time (min) necessary for a sample to swell to 70 vol% with respect to the saturated swelling degree by absorbing physiological saline, and it is measured by the following method.

<Method for measuring the absorption time (Z)>

1.0 g of a sample is put in a 100 ml measuring cylinder, and 100 ml of physiological saline is charged further at once. A volume of the swelling sample is read every one minute (a marking of the measuring cylinder corresponding to the top end of the swelling sample is read as indicating the volume of the sample), and the measurement is carried out continuously until a change in the measured value that has occurred during 5 minutes becomes± 0 (the sample gradually swells while absorbing physiological saline, whereby its volume is increasing, and the sample assumes a state of saturated swelling after a certain period of time). A volume-time graph is plotted in which the traverse axis shows the time and the longitudinal axis shows the volume of the sample thus swelling. A volume measured when the change in the measured value during 5 minutes becomes ±0 (saturated swelling degree) is assumed to be 100 percent by volume (vol%), and a time corresponding to 70 vol% with respect to the foregoing saturated swelling degree is assumed to be an absorption time (Z).

It should be noted that temperature of physiological saline used and ambient atmosphere for the measurement is set to 25°C ±2°C.

Preferably formula (12) is satisfied instead of formula (3), more preferably formula (13) is satisfied, particularly preferably formula (14) is satisfied, and most preferably formula (15) is satisfied. By satisfying such a formula, an absorbent article that exhibits a further improved absorption performance under any condition and is substantially free from leakage can be produced more easily.

$$(Z) \leq -0.0071\,(Y) + 2.8 \qquad (12)$$

$$(Z) \leq -0.0071\,(Y) + 2.5 \qquad (13)$$

$$(Z) \leq -0.0071\,(Y) + 2.3 \qquad (14)$$

$$(Z) \leq -0.0071\,(Y) + 2.0 \qquad (15)$$

Further, in formula (3), preferably formula (16) is satisfied, more preferably formula (17) is satisfied, particularly preferably formula (18) is satisfied, and most preferably formula (19) is satisfied. By satisfying such a formula, an absorbent article that exhibits a further improved absorption performance under any condition and is substantially free from leakage can be produced more easily.

$$1 \leq (Y) \leq 80 \qquad (16)$$

$$5 \leq (Y) \leq 60 \qquad (17)$$

$$10 \leq (Y) \leq 50 \qquad (18)$$

$$15 \leq (Y) \leq 40 \qquad (19)$$

The water content in the absorbent resin particle of the present invention preferably is 1 wt% to 12 wt% from the viewpoints of workability, feeling, moisture resistance, etc. in the case where it is used in an absorbent article. More preferably, the content is 2 wt% to 10 wt%, and particularly preferably 4 wt% to 8 wt%. With the content in such a range, an absorbent resin particle is prevented from being damaged by impact, and a further improved workability and the like are obtained.

It should be noted that water content is determined not solely in the drying process, but is adjusted in the surface linking process, the water-adding process, and the like. Further, the water content can be measured by a weight decrease ratio after the drying process in the same manner (120±5°C, 30 min, etc.) as that for the above-described measurement of the moisture.

An additive may be added to an absorbent resin particle of the present invention as required at an arbitrary stage {during the polymerization process for forming the crosslinked polymer (A), the crushing process, the drying process, the pulverizing process, and/or the surface crosslinking process, and/or before or after such a process, etc.}.

Examples of the additive to be used include antiseptic agents, fungicides, antibacterial agents, antioxidants, ultraviolet absorbers, coloring agents, perfuming agents, deodorizers, organic fibrous materials, and the like. The above-mentioned materials may be used singly or in combination of two or more kinds.

Examples of antiseptics include preservatives such as salicylic acid, sorbic acid, dehydroacetic acid, methyl naphthoquinone, etc., and sterilizers such as chloramine B, nitrofurazone, etc.

Examples of fungicide include butyl p-oxybenzoate.

Examples of antibacterial agent include benzalkonium chloride salt, and chlorhexidine gluconate.

Examples of antioxidant include:

hindered phenol type antioxidant such as triethyleneglycol-bis-[3-(3-t-butyl-5-methyl-4-hydroxyphenyl) propionate], 1,6-hexanediol-bis[3-(3,5-di-t-butyl-4-hydroxyphenyl) propionate], octadecyl-3-(3,5-di-t-butyl-4-hydroxyphenyl) propionate, 3,5-di-t-butyl-4-hydroxybenzyl phosphonate-diethyl ester, etc.; and
amine type antioxidant such as n-butyl amine, triethyl amine, diethyl aminomethyl methacrylate, etc.

Examples of ultraviolet absorber include:

benzotriazole type ultraviolet absorber such as 2-(5-methyl-2-hydroxyphenyl) benzotriazole, 2-(3,5-di-t-butyl-2-hydroxyphenyl) benzotriazole, 2-(3,5-di-t-butyl-2-hydroxyphenyl)-5-chloro-benzotriazole, 2-(3,5-di-t-amyl-2-hydroxyphenyl) benzotriazole;
triazine type ultraviolet absorber such as 2-(4, 6-diphenyl-1, 3, 5-triazine-2-yl)-5- [(hexyl) oxy]-phenol, etc.; and
benzophenone type ultraviolet absorber such as 2-hydroxy-4-n-octyloxybenzophenone; and
oxalic acid anilide type ultraviolet absorber such as 2-ethoxy-2'-ethyl oxalic acid bisanilide.

Examples of coloring agent include: an inorganic pigment such as titanium oxide, ferrite, etc.; an organic pigment such as azolake type, benzimidazolone type, phthalocyanine type, etc.; and
dyes such as nigrosine type, aniline type, etc.

Examples of perfuming agent include: natural perfume such as musk, abies oil, turpentine oil, etc.; and synthetic perfume such as menthol, citral, p-methyl acetophenone, floral, etc.

Examples of deodorizer include zeolite, silica, flavonoid, cyclodextrin, etc.

When such an additive is added, the added amount of the same differs depending upon applications of use, but based on the weight of the absorbent resin particle, the amount is preferably $10^{-6}$ wt% to 20 wt%; more preferably $10^{-5}$ wt% to 10 wt%; and particularly preferably $10^{-4}$ wt% to 5 wt%. If an additive is added in such a range, antimicrobial effect etc. can be imparted to the absorbent resin particle, without lowering the absorption performance thereof.

When the absorbent resin particle of the present invention is used for various kinds of absorbers, absorbing articles excellent in the absorbing performance can be produced.

Examples of a method for applying the absorbent resin particle to an absorber include: (1) a method of dispersing particles of the absorbent resin particle between layers of fibrous materials made of pulp, etc. disposed in a layered structure; (2) a method of mixing a fibrous material including pulp, heat bondable fibers, etc. and particles of the absorbent resin particle; and (3) a method of interposing the particles of the absorbent resin particle, together with a fibrous material if necessary, between two or more pieces of water absorption paper or non-woven fabrics.

As a fibrous material, any of fibrous materials conventionally used for absorbent articles, such as various kinds of fluff pulp, cotton pulp, etc., can be used. Raw materials of the fibrous materials (needle-leaved tree, broad-leaved tree, etc.) and the production method [kraft pulp, chemical pulp, semichemical pulp, chemithermo-mechanical pulp (CTMP), etc.], bleaching method, etc. are not particularly limited. Furthermore, as a fibrous material, apart from the above-mentioned organic fibrous materials, a synthetic fiber that is not swollen with water also can be used as required singly or in combination with the above-mentioned fluff pulp, cotton pulp, etc. Examples of the synthetic fiber include

polyolefin fiber (polyethylene fiber, polypropylene fiber, etc.), polyester fiber (polyethylene terephthalate fiber, etc.), polyolefin-polyester conjugated fiber, polyamide fiber, polyacrylonitrile fiber, and the like.

The length and thickness of the fibrous material are not particularly limited. In general, the length is suitably 1 mm to 200 mm and the thickness is suitably 0.1 denier to 100 denier (0.11 dtex to 110 dtex).

The shape is not particularly limited as long as it is in a fibrous form. Examples of the form of the fiber include a web form, a thin cylindrical form, a cut split yarn form, a staple form, a filament form, and the like.

The added amount of the absorbent resin particle of the present invention with respect to the absorber can be altered depending upon the kind or size of the absorber, and absorption performance to be obtained. However, the amount is preferably 30 wt% to 95 wt%, more preferably 40 wt% to 94 wt%, and particularly preferably 50 wt% to 93 wt% based on the total weight of the absorbent resin particle and fibrous material. When the amount is in such a range, a further improved absorption performance of the obtained absorber can be obtained.

An absorber in which an absorbent resin particle of the present invention is employed can provide a dry feeling even if it absorbs liquid (body fluid such as sweat, urine, or blood, water such as seawater, underground water, or muddy water, etc.). Therefore, when the absorber is applied to hygienic products such as disposable diapers, sanitary napkins, etc., the hygienic products exhibit not only excellent absorption performance but also an excellent property in preventing absorbed liquid from returning easily even under pressure.

Accordingly, by using the absorbent resin particle of the present invention, it is possible to readily manufacture absorbent articles that exhibit high absorption performance under any condition.

That is to say, even in the case where the absorbent articles are in a state under loading, for example, where a user wearing the absorbent article sits down or lies down, the absorption amount and the absorption rate are not deteriorated. As a result, the problems such as leakage and the like hardly occur.

As an absorbent article, an absorbent article composed of an absorber, a liquid permeable sheet, and an air permeable back sheet is preferable. More preferable example is an absorbent article as a hygienic article.

Examples of hygienic articles include disposable diapers (disposable diapers for children, disposable diapers for adults, etc.), napkin (sanitary napkin, etc.), paper towel, pads (incontinence pads, underpads for surgical operations, etc.), pet sheets (pet urine absorption sheet), and the like. The absorbent article is more suitable for use in disposable diapers among the foregoing hygienic products. Furthermore, among disposable diapers, the absorbent article is most suitable for use in disposable diapers that are required to exhibit a surface dryness value of preferably 50% or more, more preferably 55% or more, measured by the following SDME method.

<Surface dryness value by SDME method>

The surface dryness value according to the SDME method is measured in the following procedure by using a SDME (Surface Dryness Measurement Equipment) tester (manufactured by WK System Co.).

The detector of the SDME tester is placed on a sufficiently wetted disposable diaper (the disposable diaper had been soaked in artificial urine (0.03 wt% of calcium chloride, 0.08 wt% of magnesium sulphate, 0.8 wt% of sodium chloride, and 99.09 wt% of ion-exchanged water) in an amount of covering disposable diaper and left to stand still for 60 minutes), and a value obtained therefrom is assumed to be a dryness value of 0%. Next, the detector of the SDME tester is placed on a dry disposable diaper (disposable diaper was heated and dried at 80˚C for 2 hours), and a value obtained therefrom is assumed to be a dryness value of 100%. Thus, the adjustment of the SDME tester is carried out.

Next, a metallic ring (internal diameter: 70 mm, external diameter: 80 mm, length: 50 mm, and weight: 300 g) is set in the center of a disposable diaper to be measured and 80 ml of artificial urine is poured. Immediately after pouring, the metallic ring is removed and the SDME detector is set in contact with the disposable diaper in the center thereof and measurement is started. The value detected 5 minutes after the start of the measurement is defined as a surface dryness value according to the SDME.

It should be noted that the absorbent resin particle of the present invention is useful not only for use in the above-mentioned hygienic products but also for various applications such as a pet urine absorbing agent, a urine gelling agent for portable toilet, a freshness retaining agent for vegetables and fruits, a drip absorbing agent for meat and fish, a cold-retaining material, a disposable pocket warmer, a gelling agent for batteries, a water retention material for plants and soil, etc., an anti-dewing material, a water sealing agent, a packing agent, artificial snow, etc.

## Example

Hereinafter, the present invention will be described in more detail by way of Examples and Comparative Examples. However, the present invention is not necessarily limited thereto. Hereinafter, "part" means "part by weight" and "%" means "wt%" unless otherwise specified.

<Synthesis of hydrogel-form polymer (AA-1)>

77 parts of sodium acrylate, 22.85 parts of acrylic acid, 0.15 part of N,N'-methylene bisacrylamide, 293 parts of deionized water, and 0.001 part of dichlorotris(triphenylphosphine)ruthenium were placed in a glass reaction container and the contents were mixed and stirred while being maintained at 3˚C.

After introducing nitrogen into the contents so as to make the dissolved oxygen content 1 ppm or less, 0.3 part of 1 % aqueous solution of hydrogen peroxide, 0.8 part of 0.2 % aqueous solution of ascorbic acid, and 0.8 parts of 2 % aqueous solution of 2,2'-azobisamidinopropane dihydrochloride were added and mixed for initializing the polymerization. After the temperature of the reaction solution reached 80˚C, polymerization was allowed to occur at the temperature of 80 $\pm$ 2˚C for about 5 hours. As a result, a hydrogel-form polymer (AA-1) formed with a crosslinked polymer (A1) was obtained. It had a water content of 74.6 % (120$\pm$5˚C $\times$ 30 min).

<Preparation of material (DD11)>

0.0005 part of amino-modified silicone ("KF354" produced by Shin-Etsu Chemical Co., Ltd.) was dissolved in 10 parts of methanol, and the solution thus obtained was added to 5 parts of clay ("LAPONIPEXLG" produced by ROCKWOOD ADDITIVES Ltd.). The solution thus obtained was stirred for 2 minutes at 25˚C by a biomixer ("ABM-2" manufactured by NISSEI Corporation), and subsequently was dried at 60˚C for one hour, whereby a hydrophobicity imparted material (DD11) was obtained.

<Preparation of material (DD12)>

0.0005 part of dimethyl silicone (trade name: "SH200", produced by Dow Corning Toray Silicone Co., Ltd.) was dissolved in 10 parts of methanol, and the solution thus obtained was added to 5 parts of pulp ("KC Flock W-400G" produced by Nippon Paper Chemicals, Co., Ltd.). The solution thus obtained was stirred for 2 minutes at 25˚C by a biomixer ("ABM-2" manufactured by NISSEI Corporation), and subsequently was dried at 60˚C for one hour, whereby a hydrophobicity imparted material (DD12) was obtained.

<Preparation of material (DD13)>

0.0005 part of dimethyl silicone (trade name: "SH200", produced by Dow Corning Toray Silicone Co., Ltd.) was dissolved in 10 parts of methanol, and the solution thus obtained was added to 5 parts of silica ("Aerosil 200PE" produced by NIPPON AEROSIL Co., Ltd.). The solution thus obtained was stirred for 2 minutes at 25˚C by a biomixer ("ABM-2" manufactured by NISSEI Corporation), and subsequently was dried at 60˚C for one hour, whereby a hydrophobicity imparted material (DD13) was obtained.

<Preparation of material (DD14)>

0.0005 part of liquid paraffin (viscosity: 50 mPa·s) was dissolved in 10 parts of cyclohexane, and the solution thus obtained was added to 5 parts of clay ("LAPONIPE XLG" produced by ROCKWOOD ADDITIVES Ltd.). The solution thus obtained was stirred for 2 minutes at 25˚C by a biomixer ("ABM-2" manufactured by NISSEI Corporation), and subsequently was dried at 60˚C for one hour, whereby a hydrophobicity imparted material (DD14) was obtained.

<Preparation of material (DD15)>

0.0005 part of carboxyl-modified wax (trade name: "SANWAX 165-P", produced by Sanyo Chemical Industries Co., Ltd.) was dissolved in 10 parts of cyclohexane, and the solution thus obtained was added to 5 parts of pulp ("KC Flock W-400G" produced by Nippon Paper Chemicals, Co., Ltd.). The solution thus obtained was stirred for 2 minutes at 25˚C by a biomixer ("ABM-2" manufactured by NISSEI Corporation), and subsequently was dried at 60˚C for one hour, whereby a hydrophobicity imparted material (DD15) was obtained.

<Preparation of material (DD16)>

0.0005 part of carboxyl-modified wax (trade name: "SANWAX 165-P", produced by Sanyo Chemical Industries Co., Ltd.) was dissolved in 10 parts of cyclohexane, and the solution thus obtained was added to 5 parts of silica ("Aerosil 200PE" produced by NIPPON AEROSIL Co., Ltd.). The solution thus obtained was stirred for 2 minutes at 25˚C by a biomixer, and subsequently was dried at 60˚C for one hour, whereby a hydrophobicity imparted material (DD16) was obtained.

<Example 1>

40 parts of the material (DD11) and 0.4 part of a diffusing-penetrating agent (E1) (anionic surface active agent "SANMORIN OT70" produced by Sanyo Chemical Industries Co., Ltd.) were added to 400 parts of the hydrogel-form polymer (AA-1). The obtained mixture was kneaded for 5 minutes at 25˚C by a mincing machine (diameter of perforation of perforated plate: 6 mm, "12VR-400K" manufactured by Iizuka Kogyo K. K.), and subsequently dried by a through-flow band type drier under the conditions of a temperature of 135˚C and a wind speed of 2.0 m/sec so as to obtain a dry polymer.

This dry polymer was pulverized by using a commercially available juicer mixer, and the particle size was adjusted to 250 μm to 600 μm by using sieves with 600 μm and 250 μm mesh openings. 100 parts of the particles thus obtained were stirred at high speed (by using "High-speed stirring turbulizer" manufactured by Hosokawa Micron Co., rate of revolution; 2000 rpm) while adding by spraying 2 parts of 10% water/methanol mixture solution of ethylene glycol diglycidyl ether (weight ratio of water / methanol = 70 / 30) to be mixed. The mixture was allowed to stand at 140˚C for 30 minutes to be crosslinked by heating, and thereby an absorbent resin particle (1) was obtained.

<Example 2>

An absorbent resin particle (2) was obtained in the same manner as that of Example 1 except that the material (DD12) was used in place of the material (DD11).

<Example 3>

An absorbent resin particle (3) was obtained in the same manner as that of Example 1 except that the material (DD 13) was used in place of the material (DD11).

<Example 4>

An absorbent resin particle (4) was obtained in the same manner as that of Example 1 except that the material (DD14) was used in place of the material (DD11).

<Example 5>

An absorbent resin particle (5) was obtained in the same manner as that of Example 1 except that the material (DD15) was used in place of the material (DD11).

<Example 6>

An absorbent resin particle (6) was obtained in the same manner as that of Example 1 except that the material (DD16) was used in place of the material (DD11).

<Example 7>

An absorbent resin particle (7) was obtained in the same manner as that of Example 1 except that a diffusing-penetrating agent (E2) (non-ionic surface active agent "NAROACTY ID50" produced by Sanyo Chemical Industries Co., Ltd.) was used in place of the diffusing-penetrating agent (E1) ("SANMORIN OT70" produced by Sanyo Chemical Industries Co., Ltd.).

<Example 8>

An absorbent resin particle (8) was obtained in the same manner as that of Example 1 except that the material (DD12) was used in place of the material (DD11) and the diffusing-penetrating agent (E2) ("NAROACTY ID50" produced by Sanyo Chemical Industries Co., Ltd.) was used in place of the diffusing-penetrating agent (E1) ("SANMORIN OT70" produced by Sanyo Chemical Industries Co., Ltd.).

<Example 9>

An absorbent resin particle (9) was obtained in the same manner as that of Example 1 except that the weight of the material (DD11) was altered to 5.4 parts.

<Example 10>

An absorbent resin particle (10) was obtained in the same manner as that of Example 1 except that the used amount, 40 parts, of the material (DD11) was altered to 2.7 parts, and the used amount, 0.4 part, of the diffusing-penetrating agent (E1) ("SANMORIN OT70" produced by Sanyo Chemical Industries Co., Ltd.) was altered to 0.14 part.

<Example 11>

40 parts of silicone beads ("TOSPEARL" manufactured by GE Toshiba Silicones, average particle diameter: 2 μm) and 0.4 part of the diffusing-penetrating agent (E2) ("NAROACTY ID50" produced by Sanyo Chemical Industries Co., Ltd.) were added to 400 parts of the hydrogel-form polymer (AA-1), and mixed and kneaded for 5 minutes at 25˚C by a mincing machine (diameter of perforation of perforated plate: 6 mm, "12VR-400K" manufactured by Iizuka Kogyo K. K.). The obtained mixture was subsequently dried by a through-flow band type drier under the conditions of a temperature of 135˚C and a wind speed of 2.0 m/sec so as to obtain a dry polymer.

This dry polymer was pulverized by using a commercially available juicer mixer, and the particle size was adjusted to 250 μm to 600 μm by using sieves with 600 μm and 250 μm mesh openings. 100 parts of the particles thus obtained were stirred at high speed (by using "High-speed stirring turbulizer" manufactured by Hosokawa Micron Co., rate of revolution; 2000 rpm) while adding by spraying 2 parts of 10% water/methanol mixture solution of ethylene glycol diglycidyl ether (weight ratio of water / methanol = 70 / 30) to be mixed. The mixture was allowed to stand at 140˚C for 30 minutes to be crosslinked by heating, and thereby an absorbent resin particle (11) was obtained.

<Example 12>

An absorbent resin particle (12) was obtained in the same manner as that of Example 11 except that a material obtained by crushing a polyethylene film (polyethylene film produced by TAMAPOLY Co., Ltd., thickness: 30 μm, passed through a 250-μm-opening mesh) was used in place of the silicone beads.

<Example 13>

40 parts of a wax emulsion (trade name: "CHEMIPEARL S650", manufactured by Mitsui Chemicals, Inc.) and 0.4 part of the diffusing-penetrating agent (E2) ("NAROACTY ID50" produced by Sanyo Chemical Industries Co., Ltd.) were added to 400 parts of the hydrogel-form polymer (AA-1). The obtained mixture was kneaded for 5 minutes at 25˚C by a mincing machine (diameter of perforation of perforated plate: 6 mm, "12VR-400K" manufactured by Iizuka Kogyo K. K.), and subsequently dried by a through-flow band type drier under the conditions of a temperature of 135˚C and a wind speed of 2.0 m/sec so as to obtain a dry polymer.

This dry polymer was pulverized by using a commercially available juicer mixer, and the particle size was adjusted to 30 mesh to 60 mesh by using sieves with 590 μm and 250 μm mesh openings. 100 parts of the particles thus obtained were stirred at high speed (by using "High-speed stirring turbulizer" manufactured by Hosokawa Micron Co., rate of revolution; 2000 rpm) while adding by spraying 2 parts of 10% water/methanol mixture solution of ethylene glycol diglycidyl ether (weight ratio of water / methanol = 70 / 30) to be mixed. The mixture was allowed to stand at 140˚C for 30 minutes to be crosslinked by heating, and thereby an absorbent resin particle (13) was obtained.

<Example 14>

An absorbent resin particle (14) was obtained in the same manner as that of Example 13 except that the used amount of the wax emulsion, 40 parts, was altered to 1.7 parts.

<Example 15>

An absorbent resin particle (15) was obtained in the same manner as that of Example 13 except that the used amount of the wax emulsion, 40 parts, was altered to 0.14 parts, and the used amount of the diffusing-penetrating agent (E2) ("SANMORIN OT70" produced by Sanyo Chemical Industries Co., Ltd.), 0.4 part, was altered to 0.14 part.

<Example 16>

77 parts of sodium acrylate, 22.85 parts of acrylic acid, 0.15 part of N,N'-methylenebis acrylamide, 293 parts of deionized water, 0.001 part of dichlorotris(triphenylphosphine)ruthenium, and 0.2 part of a hydrophobic substance (C11) (non-ionic surface active agent, "NAROACTY ID20" produced by Sanyo Chemical Industries Co., Ltd.) were

charged in a glass reaction container and the contents were stirred and mixed while the temperature thereof was maintained at 3°C.

After introducing nitrogen into the contents so as to make the dissolved oxygen content 1 ppm or less, 0.3 part of 1 % aqueous solution of hydrogen peroxide, 0.8 part of 0.2 % aqueous solution of ascorbic acid; and 0.8 parts of 2 % aqueous solution of 2,2'-azobisamidinopropane dihydrochloride were added and mixed for initializing the polymerization. After the temperature of the reaction solution reached 80°C, polymerization was allowed to occur at the temperature of 80 $\pm$ 2°C for about 5 hours. As a result, a hydrogel-form polymer (AA-2) formed with a crosslinked polymer (A2) was obtained. 0.4 part of the diffusing-penetrating agent (E2) ("NAROACTY ID50" produced by Sanyo Chemical Industries Co., Ltd.) was added to 400 parts of the hydrogel-form polymer (AA-2). The obtained mixture was kneaded for 5 minutes at 25°C by a mincing machine (diameter of perforation of perforated plate: 6 mm, "12VR-400K" manufactured by Iizuka Kogyo K. K.), and subsequently dried by a through-flow band type drier under the conditions of a temperature of 135°C and a wind speed of 2.0 m/sec so as to obtain a dry polymer.

This dry polymer was pulverized by using a commercially available juicer mixer, and the particle size was adjusted to 250 μm to 600 μm by using sieves with 600 μm and 250 μm mesh openings. 100 parts of the particles thus obtained were stirred at high speed (by using "High-speed stirring turbulizer" manufactured by Hosokawa Micron Co., rate of revolution; 2000 rpm) while adding by spraying 2 parts of 10% water/methanol mixture solution of ethylene glycol diglycidyl ether (weight ratio of water / methanol = 70 / 30) to be mixed. The mixture was allowed to stand at 140°C for 30 minutes to be crosslinked by heating, and thereby an absorbent resin particle (16) was obtained.

<Example 17>

145.4 parts of acrylic acid was diluted with 9.4 parts of water, and was neutralized by adding thereto 242.3 parts of 25 % aqueous solution of sodium hydroxide while being cooled to 30°C to 20°C. To the solution thus obtained, 0.09 part of ethylene glycol diglycidyl ether ("Denacol EX-810" produced by Nagase ChemteX Corporation), 0.0146 part of sodium hypophosphite monohydrate, 0.0727 part of potassium persulfate, and 0.57 part of the diffusing-penetrating agent (E1) ("SANMORIN OT70" produced by Sanyo Chemical Industries Co., Ltd.) were added so as to be dissolved. 8.5 parts of the material (DD15) was added thereto and stirred for 2 minutes at 25°C by a biomixer ("ABM-2" manufactured by NISSEI Corporation) so as to be dispersed, whereby an aqueous solution of monomer was obtained. Subsequently, 624 parts of cyclohexane was placed in a reaction container equipped with an agitator, a reflux condenser, a thermometer, and a nitrogen gas charging tube, and 1.56 parts of polyoxyethylene octyl phenyl ether phosphoric ester (trade name: "PLYSURF A210G" manufactured by Dai-Ichi Kogyo Seiyaku Co., Ltd) was added and dissolved therein. The mixture was stirred and-subjected to nitrogen gas substitution, and thereafter was heated to 70°C. While the temperature was maintained at 70°C, the foregoing prepared aqueous solution of monomer was dropped thereto at a rate of 6.6 parts/min for 6 minutes, and was maintained at 75°C for 15 minutes. Thereafter, the remaining aqueous solution of monomer was dropped thereto at a rate of 6.6 parts/min for 54 minutes. Then, it was aged at 75°C for 30 minutes. Thereafter, water was removed by azeotropy with cyclohexane so that the water content in the resin became approximately 20 % (measured by an infrared water content measuring machine ("FD-100" manufactured by KETT Co.), 180°C, 20 minutes). After cooling to 30°C, the stirring was stopped, whereby resin particles settled. The resin particles were separated from cyclohexane by decantation. 80 parts of the resin particles and 140 parts of cyclohexane were put in the reaction container, and 3.4 parts of cyclohexane solution containing 0.4 % of glycerin polyglycidyl ether (trade name: "Denacol EX-314", produced by Nagase ChemteX Corporation) were added thereto. Thereafter, the mixture was maintained while being heated at 60°C for 30 minutes, and was maintained further under reflux of cyclohexane for 30 minutes while being heated. Subsequently, the mixture was filtered so that the resin particles were obtained, which were dried under reduced pressure at 80°C. As a result, an absorbent resin particle (17) was obtained.

<Example 18>

An absorbent resin particle (18) was obtained in the same manner as that of Example 17 except that the material (DD16) was used in place of the material (DD15).

<Example 19>

An absorbent resin particle (19) was obtained in the same manner as that of Example 17 except that a wax emulsion (trade name: "CHEMIPEARL S650", manufactured by Mitsui Chemicals, Inc.) was used in place of the material (DD15).

<Example 20> .

An absorbent resin particle (20) was obtained in the same manner as that of Example 17 except that the used amount of the material (DD15), 8.5 parts, was altered to 3.5 parts, and the used amount of the diffusing-penetrating agent (E1) ("SANMORIN OT70" produced by Sanyo Chemical Industries Co., Ltd.), 0.57 part, was altered to 0.17 part.

<Comparative Example 1>

A comparative absorbent resin particle (1') was obtained in the same manner as that of Example 1 except that the material (D) and the diffusing-penetrating agent (E1) were not added to the hydrogel-form polymer (AA-1).

<Comparative Example 2>

A comparative absorbent resin particle (2') was obtained in the same manner as that of Example 1 except that only the diffusing-penetrating agent (E1) were not added to the hydrogel-form polymer (AA-1).

<Comparative Example 3>

A comparative absorbent resin particle (3') was obtained in the same manner as that of Example 1 except that pulp ("KC Flock W-400G" produced by Nippon Paper Chemicals Co., Ltd.) was used in place of the material (DD12).

<Comparative Example 4>

A comparative absorbent resin particle (4') was obtained in the same manner as that of Example 1 except that silica ("Aerosil 200PE" produced by NIPPON AEROSIL Co., Ltd.) was used in place of the material (DD13).

<Comparative Example 5>

A comparative absorbent resin particle (5') was obtained in the same manner as that of Example 17 except that the material (DD15) and the diffusing-penetrating agent (E1) ("SANMORIN OT70") were not added.

<Comparative Example 6>

A comparative absorbent resin particle (6') was obtained in the same manner as that of Example 17 except that the material (DD15) was not added.

<Comparative Example 7>

A comparative absorbent resin particle (7') was obtained in the same manner as that of Example 17 except that pulp ("KC Flock W-400G" produced by Nippon Paper Chemicals Co., Ltd.) was used in place of the material (DD15).

<Comparative Example 8>

A comparative absorbent resin particle (8') was obtained in the same manner as that of Example 17 except that silica ("Aerosil 200PE" produced by NIPPON AEROSIL Co., Ltd.) was used in place of the material (DD15).

Regarding the diffusion absorption amount (Q) {a dropping pump manufactured by Todoroki Sangyo Co., Ltd., trade name: "CP-21", was used as metering pump}, the water-retention amount (X), the liquid permeation rate under loading (Y), and the absorption time (Z) (min) necessary for a sample to swell up to 70 vol% of the saturated swelling degree by absorbing physiological saline, the absorbent resin particles (1) to (20) and the comparative absorbent resin particles (1') to (8') were evaluated by the aforementioned methods, and the results of the evaluation are shown in Tables 1 and 2 below.

Table 1

| | Example | | | | | | | | | | | | | | |
|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 |
| Absorbent resin particle | (1) | (2) | (3) | (4) | (5) | (6) | (7) | (8) | (9) | (10) | (11) | (12) | (13) | (14) | (15) |
| Diffusion absorption amount (ml) | 50 | 52 | 53 | 55 | 52 | 57 | 58 | 52 | 52 | 49 | 54 | 55 | 53 | 52 | 49 |
| Water-retention amount (X) (g/g) | 36 | 36 | 35 | 35 | 35 | 35 | 36 | 36 | 35 | 34 | 35 | 35 | 36 | 33 | 36 |
| Liquid permeation rate under loading (Y) (ml/min) | 12 | 15 | 15 | 16 | 20 | 24 | 21 | 19 | 19 | 12 | 19 | 20 | 15 | 12 | 12 |
| Absorption time (Z) (min) | 1.8 | 1.6 | 1.2 | 1.1 | 1.7 | 1.0 | 1.1 | 1.1 | 1.3 | 1.0 | 1.2 | 0.9 | 0.9 | 0.9 | 1.8 |
| -0.0071(Y) +2.7 | 2.4 | 2.4 | 2.5 | 2.5 | 2.5 | 2.5 | 2.4 | 2.4 | 2.5 | 2.4 | 2.5 | 2.5 | 2.4 | 2.4 | 2.4 |

Table 2

| | Example | | | | | Comparative Example | | | | | | | |
|---|---|---|---|---|---|---|---|---|---|---|---|---|---|
| | 16 | 17 | 18 | 19 | 20 | 1 | 2 | 3 | 4 | 5 | 6 | 7 | 8 |
| Absorbent resin particle | (16) | (17) | (18) | (19) | (20) | (1') | (2') | (3') | (4') | (5') | (6') | (7') | (8') |
| Diffusion absorption amount (ml) | 52 | 52 | 53 | 55 | 50 | 37 | 40 | 23 | 37 | 35 | 39 | 35 | 36 |
| Water-retention amount (X) (g/g) | 36 | 35 | 36 | 35 | 36 | 34 | 34 | 34 | 35 | 32 | 32 | 34 | 35 |
| Liquid permeation rate under loading (Y) (ml/min) | 15 | 14 | 15 | 20 | 12 | 11 | 12 | 13 | 11 | 8 | 8 | 9 | 9 |
| Absorption time (Z) (min) | 1.2 | 0.9 | 0.9 | 0.9 | 1.8 | 5.0 | 3.7 | 5.1 | 5.0 | 4.8 | 4.1 | 4.5 | 4.5 |
| -0.0071(Y)+2.7 | 2.4 | 2.5 | 2.4 | 2.5 | 2.4 | 2.5 | 2.5 | 2.5 | 2.5 | 2.6 | 2.6 | 2.6 | 2.6 |

<Example 21>

A mixture obtained by mixing 100 parts of fluff pulp and 100 parts of the absorbent resin particle (1) obtained in Example 1 of the present invention by using an air blender was piled evenly so as to have a basis weight of about 400 $g/m^2$, and pressed with the pressure of 5 $Kg/cm^2$ for 30 seconds to obtain an absorber (B1) of Example 21. The obtained absorber (B1) was cut into a rectangular shape of 14 cm × 36 cm, and on the upper surface and the lower surface of the absorber having the rectangular shape, sheets of water absorbing paper (basis weight: 15.5 $g/m^2$) having the same size as that of the absorber (B1) were placed, respectively. Furthermore, a polyethylene sheet used in a commercially available disposable diaper was placed on a rear side and a polyethylene non-woven fabric (basis weight:

20.0 g/m$^2$) was placed on a front side, whereby a disposable diaper (1) was produced.

<Examples 22 to 40>

Likewise, absorbers (B2) to (B20) were prepared by using the absorbent resin particles (2) to (20), respectively, in place of the absorbent resin particle (1), and further, disposable diapers (2) to (20) were produced with the same, respectively.

<Example 41>

After a layer was formed with 50 parts of fluff pulp, 100 parts of the absorbent resin particle (2) obtained in Example 2 was dispersed thereon evenly, and further a layer formed with 50 parts of fluff pulp was stacked... thereon, whereby a sandwich structure was obtained. The structure was pressed with the pressure of 5 Kg/cm$^2$ for 30 seconds to obtain an absorber (B21). The obtained absorber (B21) was cut into a rectangular shape of 14 cm × 36 cm, and on the upper surface and the lower surface of the absorber having the rectangular shape, sheets of water absorbing paper (basis weight: 15.5 g/m$^2$) having the same size as that of the absorber were placed, respectively. Furthermore, a polyethylene sheet used in a commercially available disposable diaper was placed on a rear side and a polyethylene non-woven fabric (basis weight: 20.0 g/m$^2$) was placed on a front side, whereby a disposable diaper (21) was produced.

<Comparative Examples 9 to 16>

Comparative absorbers (B1') to (B8') were prepared in the same manner as that of Example 21 except that the comparative absorbent resin particles (1') to (8') obtained in Comparative Examples 1 to 8 were used, respectively, in place of the absorbent resin particle (1), and further, disposable diapers (1') to (8') were produced with the same, respectively.

An absorption amount until leakage and a surface dry feeling of each of the disposable diapers (1) to (21) and (1') to (8') thus formed were measured by the following methods. A surface dryness value according to SDME of each diaper was measured by the above-described method. The results of the measurement are shown in Table 3.

<Absorption amount until leakage>

Each disposable diaper (140 mm × 360 mm) was placed on an acryl plate (140 mm × 360 mm, weight: 0.5 Kg), and an end (upper end) of one shorter side (140 mm) of the disposable diaper was fixed to the acryl plate with a gummed tape (width of an overlapping allowance of the disposable diaper and the gummed tape: 1 cm from the end). The acryl plate was fixed in a tilted state with a tilt of 45°, in a manner such that the end at which the disposable diaper was fixed (upper end) was disposed on the upper side. Then, at a location of 30 mm from the upper end {330 mm from the other end (lower end)} and 70 mm from both longer sides, artificial urine (0.03 wt% of calcium chloride, 0.08 wt% of magnesium sulphate, 0.8 wt% of sodium chloride, and 99.09 wt% of ion-exchanged water) was charged by a dropping pump (manufactured by Todoroki Sangyo Co., Ltd., trade name: "CP-21") at a rate of 100 g/min. An amount of the artificial urine charged until the artificial urine leaked from the lower end of the disposable diaper was determined, and this amount was assumed to be an absorption amount until leakage.

<Surface dry feeling>

10 persons felt with fingers a surface of each disposable diaper that had been used for determining the absorption amount until leakage so as to evaluate a surface dry feeling of the disposable diaper with the following four criteria. An average of the evaluations by the 10 persons was obtained as a surface dry feeling.

○: Excellent dry feeling
Δ: Dry feeling at a satisfactory level with slight wetting
×: Wettish state with poor dry feeling or wet state without dry feeling

Table 3

| | | Disposable diaper | Performance of disposable diaper | | |
|---|---|---|---|---|---|
| | | | Absorption amount until leakage (g) | Surface dry feeling | SDME Surface dryness value (%) |
| Example | 21 | 1 | 400 | ○ | 77 |
| | 22 | 2 | 370 | ○ | 65 |
| | 23 | 3 | 380 | ○ | 73 |
| | 24 | 4 | 400 | ○ | 67 |
| | 25 | 5 | 400 | ○ | 65 |
| | 26 | 6 | 430 | ○ | 75 |
| | 27 | 7 | 420 | ○ | 66 |
| | 28 | 8 | 390 | ○ | 57 |
| | 29 | 9 | 430 | ○ | 60 |
| | 30 | 10 | 380 | ○ | 55 |
| | 31 | 11 | 450 | ○ | 67 |
| | 32 | 12 | 430 | ○ | 69 |
| | 33 | 13 | 440 | ○ | 74 |
| | 34 | 14 | 450 | ○ | 72 |
| | 35 | 15 | 380 | ○ | 62 |
| | 36 | 16 | 430 | ○ | 67 |
| | 37 | 17 | 430 | ○ | 70 |
| | 38 | 18 | 450 | ○ | 71 |
| | 39 | 19 | 440 | ○ | 69 |
| | 40 | 20 | 410 | ○ | 62 |
| | 41 | 21 | 460 | ○ | 80 |
| Comparative Example | 9 | 1' | 320 | △ | 35 |
| | 10 | 2' | 310 | △ | 24 |
| | 11 | 3' | 310 | △ | 26 |
| | 12 | 4' | 310 | △ | 36 |
| | 13 | 5' | 320 | △ | 36 |
| | 14 | 6' | 310 | △ | 32 |
| | 15 | 7' | 350 | △ | 38 |
| | 16 | 8' | 310 | △ | 33 |

The absorbent resin particle of the present invention can be used for various absorbers, and by so doing, absorbent articles that are substantially free from leakage of absorbed liquid can be obtained. Particularly, the absorbent resin particle is suitably employed in hygienic articles such as disposable diapers (disposable diapers for children, disposable diapers for adults, etc.), napkin (sanitary napkin, etc.), paper towel, pads (incontinence pads, underpads for surgical operations, etc.), and pet sheets (pet urine absorption sheet), among which it is most suitable for use in disposable

diapers.

It should be noted that the absorbent resin particle of the present invention is useful not only for use in hygienic products but also for various applications such as a pet urine absorbing agent, a urine gelling agent for portable toilet, a freshness retaining agent for vegetables and fruits, a drip absorbing agent for meat and fish, a cold retaining material, a disposable pocket warmer, a gelling agent for batteries, a water retention material for plants and soil, etc., an anti-dewing material, a water sealing agent, a packing agent, artificial snow, etc.

## Claims

**1.** An absorbent resin particle comprising:

a crosslinked polymer (A) including, as essential constituent units, a water soluble vinyl monomer (a1) and/or a vinyl monomer (a2) that is formed into the water-soluble vinyl monomer (a1) by hydrolysis, and an internal crosslinking agent (b); and
selected from the group consisting of:

a hydrophobic substance (C1) containing a hydrocarbon group;
a hydrophobic substance (C2) containing a hydrocarbon group having a fluorine atom;
a hydrophobic substance (C3) having a polysiloxane structure;

wherein
the absorbent resin particle has a structure such that a part or an entirety of the hydrophobic substance (C) is contained in the inside of each particle of the absorbent resin particle,
particle is such that each particle of the absorbent resin particle contains a connection (RC) formed with the hydrophobic substance (C), and
the absorbent resin particle being obtainable by either one of methods (1) and (2):

the method (1) including forming the connection (RC) and thereafter mixing the hydrophobic substance (C) with a hydrogel of the crosslinked polymer (A) or a polymerization solution of the crosslinked polymer (A); and
the method (2) including forming the connection (RC) while the crosslinked polymer (A) is being produced.

**2.** The absorbent resin particle according to claim 1, wherein
the structure such that a part or an entirety of the hydrophobic substance (C) is contained in the inside of each particle of the absorbent resin particle is a structure such that a material (D) obtained by coating or impregnating a part or an entirety of either a hydrophilic material (d1) or a hydrophobic material (d2) with the hydrophobic substance (C) is contained in the inside of each particle of the absorbent resin particle.

**3.** The absorbent resin particle according claim 1 or 2, wherein the hydrophobic substance (C) has a HLB value in a range of 1 to 10.

**4.** The absorbent resin particle according claim 1 or 2, wherein the hydrophobic substance (C) is a silicone or a modified silicone.

**5.** The absorbent resin particle according any of claims 1 to 4, further comprising a diffusing-penetrating agent (E) as a constituent component.

**6.** The absorbent resin particle according to any of claims 1 to 5, wherein the absorbent resin particle exhibits a diffusion absorption amount in a range of 40 ml to 70 ml.

**7.** The absorbent resin particle according to any of claims 1 to 6, wherein
the absorbent resin particle exhibits an absorption time (Z) in a range of 0.5 minute to 3.5 minutes, the absorption time being the time necessary for the absorbent resin particle to swell to 70 percent by volume with respect to a saturated swelling degree by absorbing physiological saline.

**8.** The absorbent resin particle according to any of claims 1 to 7, wherein
the absorbent resin particle satisfies formulae (2) and (3):

$$30 \leq (X) \leq 70 \qquad (2)$$

$$(Z) \leq -0.0071(Y) + 2.7 \qquad (3)$$

where

(X) represents a water-retention amount (g/g) of the absorbent resin particle that had been immersed in physiological saline for one hour,
(Y) represents a liquid permeation rate (ml/min) under loading of 2.14kPa at which physiological saline permeates the absorbent resin particle that has been immersed in physiological saline for one hour, and
(Z) represents an absorption time (min) necessary for a sample to swell to 70 percent by volume with respect to a saturated swelling degree by absorbing physiological saline.

**9.** The absorbent resin particle according to claim 8, wherein the absorbent resin particle further satisfies formula (4):

$$10 \leq (Y) \leq 100 \qquad (4)$$

**10.** An absorber comprising:

the absorbent resin particle according to any of claims 1 to 9, and
a fibrous material.

**11.** An absorbent article comprising an absorber according to claim 10.

**Patentansprüche**

**1.** Absorbierendes Harzteilchen, umfassend:

ein vernetztes Polymer (A), welches als wesentliche Bestandteile ein wasserlösliches Vinylmonomer (a1) und/oder ein Vinylmonomer (a2), aus dem das wasserlösliche Vinylmonomer (a1) durch Hydrolyse gebildet wird, und ein internes Vernetzungsmittel (b) enthält; und
eine hydrophobe Substanz (C) ausgewählt aus:
einer hydrophoben Substanz (C1), enthaltend einen Kohlenwasserstoffrest;
einer hydrophoben Substanz (C2), enthaltend einen Kohlenwasserstoffrest mit einem Fluoratom;
einer hydrophoben Substanz (C3) mit einer Polysiloxan-Struktur;
wobei
das absorbierende Harzteilchen eine derartige Struktur aufweist, dass ein Teil oder die Gesamtheit der hydrophoben Substanz (C) im Inneren eines jeden Teilchens des absorbierenden Harzteilchens enthalten ist, derart, dass jedes Teilchen des absorbierenden Harzteilchens eine Verbindung (RC), welche mit der hydrophoben Substanz (C) gebildet ist, enthält und das absorbierende Harzteilchen durch eines der beiden Verfahren (1) und (2) erhältlich ist:
wobei das Verfahren (1) die Bildung der Verbindung (RC) und das anschließende Mischen der hydrophoben Substanz (C) mit einem Hydrogel des vernetzten Polymers (A) oder einer Polymerisationslösung des vernetzten Polymers (A) beinhaltet; und das Verfahren (2) die Bildung der Verbindung (RC), während das vernetzte Polymer (A) hergestellt wird, beinhaltet.

**2.** Absorbierendes Harzteilchen gemäß Anspruch 1, wobei die Struktur, bei der ein Teil oder die Gesamtheit der hydrophoben Substanz (C) im Inneren eines jeden Teilchens des absorbierenden Harzteilchens enthalten ist, eine derartige Struktur ist, bei der ein Material (D), welches durch Beschichtung oder Imprägnierung eines Teils oder der Gesamtheit entweder eines hydrophilen Materials (d1) oder eines hydrophoben Materials (d2) mit der hydrophoben Substanz (C) erhältlich ist, im Inneren eines jeden Teilchens des absorbierenden Harzteilchens enthalten ist.

3. Absorbierendes Harzteilchen gemäß Anspruch 1 oder 2, wobei die hydrophobe Substanz (C) einen HLB-Wert im Bereich von 1 bis 10 aufweist.

4. Absorbierendes Harzteilchen gemäß Anspruch 1 oder 2, wobei die hydrophobe Substanz (C) ein Silikon oder ein modifiziertes Silikon ist.

5. Absorbierendes Harzteilchen gemäß einem der Ansprüche 1 bis 4, weiterhin umfassend ein diffundierendes-eindringendes Mittel (E) als eine Bestandteilkomponente.

6. Absorbierendes Harzteilchen gemäß einem der Ansprüche 1 bis 5, wobei das absorbierende Harzteilchen eine Diffusionsabsorptionmenge in einem Bereich von 40 ml bis 70 ml aufweist.

7. Absorbierendes Harzteilchen gemäß einem der Ansprüche 1 bis 6, wobei das absorbierende Harzteilchen eine Absorptionszeit (Z) in einem Bereich von 0,5 Minuten bis 3,5 Minuten aufweist, wobei die Absorptionszeit die Zeit ist, die das absorbierende Harzteilchen benötigt, um auf 70 Vol.-% anzuschwellen, bezogen auf einen gesättigten Schwellungsgrad durch Absorption von physiologischer Salzlösung.

8. Absorbierendes Harzteilchen gemäß einem der Ansprüche 1 bis 7, wobei das absorbierende Harzteilchen die Formeln (2) und (3) erfüllt:

$$30 \leq (X) \leq 70 \qquad (2)$$

$$(Z) \leq -0{,}0071(Y) + 2{,}7 \qquad (3)$$

wobei

(X) eine Wasserretentionsmenge (g/g) des absorbierenden Harzteilchens, welches für eine Stunde in physiologischer Salzlösung eingetaucht wurde, darstellt,
(Y) eine Flüssigkeits-Permeationsrate (ml/min) unter einer Beladung von 2,14 kPa, bei welcher physiologische Salzlösung das absorbierende Harzteilchen, welches für eine Stunde in physiologischer Salzlösung eingetaucht wurde, permeiert, darstellt und
(Z) eine Absorptionszeit (min) darstellt, welche notwendig ist, damit eine Probe auf 70 Vol.-% anschwillt, bezogen auf einen gesättigten Schwellungsgrad durch Absorption von physiologischer Salzlösung.

9. Absorbierendes Harzteilchen gemäß Anspruch 8, wobei das absorbierende Harzteilchen weiterhin die Formel (4) erfüllt:

$$10 \leq (Y) \leq 100 \qquad (4)$$

10. Absorptionsmittel, umfassend:

das absorbierende Harzteilchen gemäß einem der Ansprüche 1 bis 9 und
ein faserförmiges Material.

11. Absorbierender Gegenstand, umfassend ein Absorptionsmittel gemäß Anspruch 10.

**Revendications**

1. Particule de résine absorbante comprenant :

un polymère réticulé (A) comprenant, comme unités constitutives essentielles, un monomère vinylique soluble

dans l'eau (a1) et/ou un monomère vinylique (a2) qui est formé dans le monomère vinylique soluble dans l'eau (a1) par hydrolyse, et un agent de réticulation interne (b); et
une substance hydrophobe (C) choisie dans le groupe comprenant :
une substance hydrophobe (C1) contenant un groupe hydrocarboné;
une substance hydrophobe (C2) contenant un groupe hydrocarboné comportant un atome de fluor;
une substance hydrophobe (C3) ayant une structure de polysiloxane;
dans laquelle :
la particule de résine absorbante a une structure telle qu'une partie ou l'entièreté de la substance hydrophobe (C) soit contenue à l'intérieur de chaque particule de la particule de résine absorbante, de telle sorte que chaque particule de la particule de résine absorbante contienne une connexion (RC) formée avec la substance hydrophobe (C), et la particule de résine absorbante étant obtenable par l'une ou l'autre des méthodes (1) et (2) :
la méthode (1) comprenant la formation de la connexion (RC) et ensuite le mélange de la substance hydrophobe (C) avec un hydrogel du polymère réticulé (A) ou une solution de polymérisation du polymère réticulé (A); et
la méthode (2) comprenant la formation de la connexion (RC) tout en produisant le polymère réticulé (A).

**2.** Particule de résine absorbante suivant la revendication 1, dans laquelle la structure telle qu'une partie ou l'entièreté de la substance hydrophobe (C) soit contenue à l'intérieur de chaque particule de la particule de résine absorbante est une structure telle qu'une matière (D) obtenue par revêtement ou imprégnation d'une partie ou de l'entièreté de l'une ou l'autre des matière hydrophile (d1) et matière hydrophobe (d2) avec la substance hydrophobe (C) soit contenue à l'intérieur de chaque particule de la particule de résine absorbante.

**3.** Particule de résine absorbante suivant la revendication 1 ou 2, dans laquelle la substance hydrophobe (C) a une valeur HLB allant de 1 à 10.

**4.** Particule de résine absorbante suivant la revendication 1 ou 2, dans laquelle la substance hydrophobe (C) est une silicone ou une silicone modifiée.

**5.** Particule de résine absorbante suivant l'une quelconque des revendications 1 à 4, comprenant de plus un agent de pénétration par diffusion (E) comme composant constitutif.

**6.** Particule de résine absorbante suivant l'une quelconque des revendications 1 à 5, dans laquelle la particule de résine absorbante montre une quantité d'absorption par diffusion allant de 40 ml à 70 ml.

**7.** Particule de résine absorbante suivant l'une quelconque des revendications 1 à 6, dans laquelle la particule de résine absorbante montre un temps d'absorption (Z) allant de 0,5 minute à 3,5 minutes, le temps d'absorption étant le temps nécessaire pour que la particule de résine absorbante gonfle de 70 % en volume par rapport au degré de gonflement saturé par absorption de solution saline physiologique.

**8.** Particule de résine absorbante suivant l'une quelconque des revendications 1 à 7, dans laquelle la particule de résine absorbante répond aux formules (2) et (3) :

$$30 \leq (X) \leq 70 \qquad (2)$$

$$(Z) \leq - 0{,}0071\ (Y) + 2{,}7 \qquad (3)$$

dans lesquelles :

(X) représente la quantité de rétention d'eau (g/g) de la particule de résine absorbante qui a été immergée dans la solution saline physiologique pendant une heure,
(Y) représente un taux de perméation de liquide (ml/min) sous un chargement de 2,14 kPa auquel la solution saline physiologique traverse la particule de résine absorbante qui a été immergée dans la solution saline physiologique pendant une heure, et
(Z) représente le temps d'absorption (minute) nécessaire pour qu'un échantillon gonfle de 70 % en volume par rapport au degré de gonflement saturé par absorption de solution saline physiologique.

**9.** Particule de résine absorbante suivant la revendication 8, dans laquelle la résine absorbante répond également à la formule (4) :

$$10 \leq (Y) \leq 100 \qquad (4)$$

**10.** Absorbant comprenant :

la particule de résine absorbante suivant l'une quelconque des revendications 1 à 9 et une matière fibreuse.

**11.** Article absorbant comprenant un absorbant suivant la revendication 10.

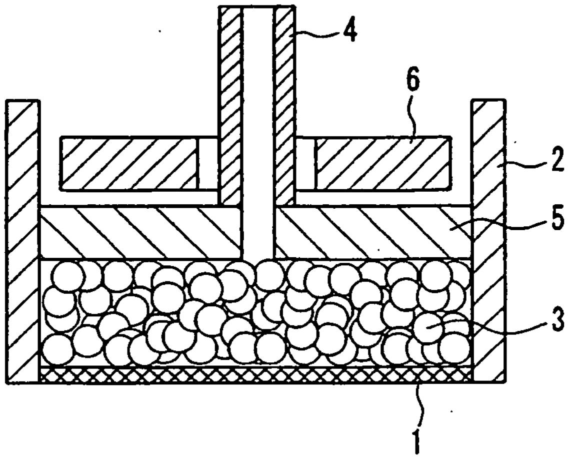


FIG. 1

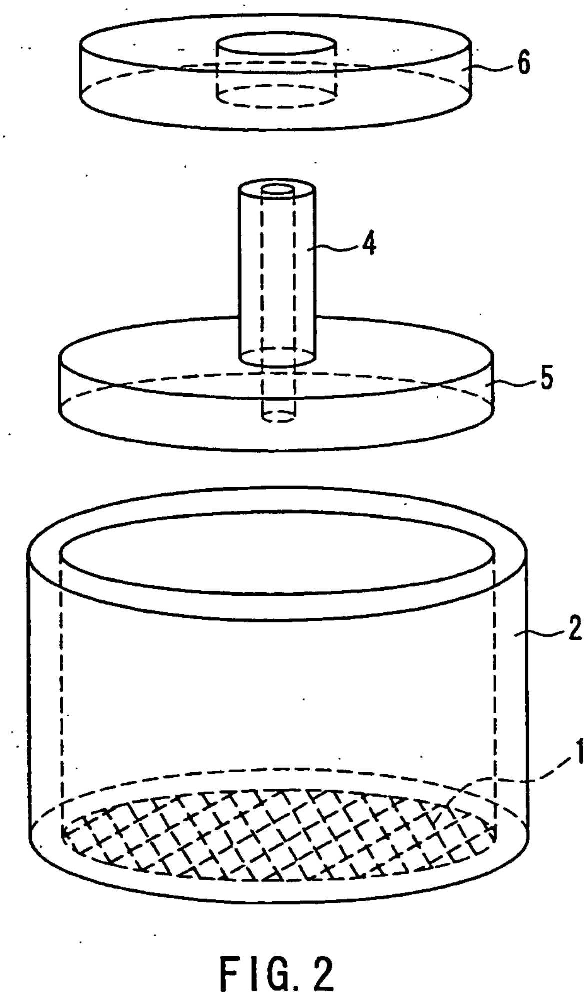


FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3179008 A
- JP 267529 B
- EP 618005 A
- JP 2002212331 A
- DE 10043710
- WO 0145758 A
- EP 463388 A
- EP 450922 A
- EP 386897 A
- US 5075373 A
- US 4732968 A
- US 4497930 A
- JP 3036966 A
- JP 58180233 A
- US 4666983 A
- JP 59189103 A


### Non-patent literature cited in the description


- **DR. TAKEHIKO FUJIMOTO.** Shin Kaimen Kassei-zai Nyumon. SANYO CHEMICAL INDUSTRIES, LTD, 1992, 132
- New Introduction to Surface Active Agents. 1985, 132
- Suiyosei Kobunshi no Saishin Gijutsu. CMC, May 2000
- Kaimen Kasseizai no Oyo Gijutsu. CMC, December 2002